# EUROPEAN PATENT APPLICATION

(11) **EP 4 610 658 A1**
(43) Date of publication of application: **03.09.2025**
(21) Application number: 25156147.8
(22) Date of filing: 06.02.2025
(51) Int. Cl.: G01N 33/68

(54) **KIT FOR QUANTITATIVE ANALYSIS OF GLYCATED ALBUMIN, METHOD FOR QUANTITATIVE ANALYSIS OF GLYCATED ALBUMIN, AND APPARATUS FOR PERFORMING THE SAME**

(30) Priority: 29.02.2024 KR 20240029436
(71) Applicant: I-SENS, INC., Seoul 06646 (KR)
(72) Inventor: Kim, Ji Hoon, 06646 Seoul (KR); Kim, Seung Wan, 06646 Seoul (KR); Park, Ji Yeon, 06646 Seoul (KR); Kim, Jin Young, 06646 Seoul (KR); Choi, Dong Cheol, 06646 Seoul (KR); Kim, Hyeong Eun, 06646 Seoul (KR)
(74) Representative: Westphal, Mussgnug & Partner Patentanwälte mbB

(57) **Abstract**

The kit for quantitative analysis of Glycated Albumin (GA) according to an embodiment of the present application comprises: a first composition including a reaction buffer for inducing binding with albumin present in a blood sample; a second composition including a decomposition reagent for a decomposition reaction to break down glycated albumin (GA) included in the blood sample into glycated amino acids; a third composition including a reaction reagent for an enzymatic reaction of the glycated amino acids present in the blood sample; and a fourth composition including a chromogenic agent being oxidized by hydrogen peroxide (H₂O₂) generated from the enzymatic reaction of the glycated amino acids.

## Description

### CROSS-REFERENCE TO RELATED APPLICATION

This application claims priority to and the benefit of Korean Patent Application No. 10-2024-0029436, filed on February 29, 2024, the disclosure of which is incorporated herein by reference in its entirety.

### BACKGROUND

### 1. Field of the Invention

The present application relates to the quantitative analysis of biological samples, and more specifically, to a kit for the quantitative analysis of glycated albumin (GA), a GA quantitative analysis method, and/or a quantitative analysis apparatus for performing the same.

### 2. Discussion of Related Art

In the diagnosis of diabetes, alongside blood glucose measurement, the need for testing glycated albumin (hereinafter referred to as GA) is increasing. Glycated albumin is a glycated substance that forms a keto-amine structure through a non-enzymatic oxidation reaction at the Lys site of albumin. The concentration of glycated albumin correlates with blood glucose levels, increasing or decreasing accordingly. Compared to HbA1c, which is widely used as an indicator for diabetes diagnosis, glycated albumin has the advantage of sensitively reflecting changes in blood glucose levels. Consequently, the development and research of technologies for quantitatively analyzing glycated albumin to diagnose diabetes are gaining significant attention.

The quantitative analysis of glycated albumin is quantified as the ratio of glycated albumin to total albumin in blood (GA%). In conventional techniques for the quantitative analysis of glycated albumin, the reaction to measure the amount of total albumin and the reaction to measure the amount of glycated albumin were separated and measured in separate spaces. This was necessarily required due to the differing reaction environments for the reaction to measure the amount of total albumin and the reaction to measure the amount of glycated albumin. According to conventional techniques, separating the two reactions resulted in limitations in miniaturization, and as a result, the quantitative analysis of glycated albumin had to be performed mainly on large biochemical equipment. Furthermore, according to conventional techniques, separating the two reactions caused the quantitative analysis of glycated albumin to take relatively longer, resulting in inconvenience to the user.

Therefore, there is a need for the development and research of technologies for the quantitative analysis of glycated albumin that can be miniaturized and improve user convenience.

### SUMMARY OF THE INVENTION

The problem to be solved by the present invention is to provide a glycated albumin quantitative analysis kit, a glycated albumin quantitative analysis method, and/or a device for performing the method, which implements the reaction for measuring the amount of total albumin and the reaction for measuring the amount of glycated albumin on a single kit, enabling miniaturization.

The problem to be solved by the present invention is to provide a glycated albumin quantitative analysis kit, a glycated albumin quantitative analysis method, and/or a device for performing the method, which implements the reaction for measuring the amount of total albumin and the reaction for measuring the amount of glycated albumin on a single kit, thereby reducing analysis time and increasing user convenience.

Objects of the present invention are not limited to the above-described objects and other objects that are not described may be clearly understood by those skilled in the art from this specification and the accompanying drawings.

The kit for quantitative analysis of Glycated Albumin (GA) according to an embodiment of the present application may comprises: a first composition including a reaction buffer for inducing binding with albumin present in a blood sample; a second composition including a decomposition reagent for a decomposition reaction to break down glycated albumin (GA) included in the blood sample into glycated amino acids; a third composition including a reaction reagent for an enzymatic reaction of the glycated amino acids present in the blood sample; and a fourth composition including a chromogenic agent being oxidized by hydrogen peroxide (H₂O₂) generated from the enzymatic reaction of the glycated amino acids, wherein the GA quantitative analysis kit may include at least one reagent fixing part, and the second composition to the fourth composition may be each independently fixed to the reagent fixing part.

Solutions of the present invention are not limited to the above-described solutions and other solutions that are not described may be clearly understood by those skilled in the art from this specification and the accompanying drawings.

### BRIEF DESCRIPTION OF THE DRAWINGS

The above and other objects, features and advantages of the present invention will become more apparent to those of ordinary skill in the art by describing exemplary embodiments thereof in detail with reference to the accompanying drawings, in which:
Fig. 1 is a schematic diagram of the glycated albumin quantitative analysis kit according to an embodiment of the present application.
Fig. 2 is a schematic diagram of the sample collector of the glycated albumin quantitative analysis kit according to an embodiment of the present application.
Fig. 3 is a schematic diagram of the main cartridge of the glycated albumin quantitative analysis kit according to an embodiment of the present application.
Fig. 4 is a schematic diagram of the solution cell of the glycated albumin quantitative analysis kit according to an embodiment of the present application.
Fig. 5 is a diagram illustrating the aspects in which the composition stored in the solution cell flows into the mixing region of the main cartridge as the sample collector is inserted into the main cartridge, according to an embodiment of the present application.
FIG 6 illustrates one aspect of an analysis method for quantifying GA according to one embodiment of the present application.
Figs. 7 and 8 are diagrams showing the specific structure of the glycated albumin quantitative analysis kit according to an embodiment of the present application.
Fig. 9 is a diagram illustrating the analysis aspect for quantifying glycated albumin in a blood sample according to an embodiment of the present application.
Fig. 10 is a table and graph showing the results of evaluating the measurement performance of albumin based on different reaction buffers, according to an embodiment of the present application.
Fig. 11 is a table and graph showing the results of evaluating the measurement performance of glycated albumin based on different reaction buffers, according to an embodiment of the present application.
Fig. 12 is a table showing the results of evaluating the measurement performance of albumin and/or glycated albumin based on surfactants added to the reaction buffer, according to an embodiment of the present application.
Fig. 13 is a table showing the results of evaluating the accelerated stability of reaction reagents, oxidases, and/or decomposition reagents based on stabilizers added thereto, according to an embodiment of the present application.
Fig. 14 is a graph showing the results of evaluating the accelerated stability of reaction reagents, oxidases, and/or decomposition reagents based on stabilizers added thereto, according to an embodiment of the present application.
Fig. 15 is a table showing the measurements and precision based on the type of disaccharide included in the stabilizers added to the reaction reagents, oxidases, and/or decomposition reagents, according to an embodiment of the present application.
Fig. 16 is a table and graph showing the results of evaluating linearity based on the type of disaccharide included in the stabilizers added to the reaction reagents, oxidases, and/or decomposition reagents, according to an embodiment of the present application.
Fig. 17 is a diagram showing the identification information included in the glycated albumin quantitative analysis kit according to an embodiment of the present application.

### DETAILED DESCRIPTION OF EXEMPLARY EMBODIMENTS

The above-described objects, features and, advantages of the present invention will be clearly understood through the following detailed description taken in conjunction with the accompanying drawings. However, while the present invention can be modified in various ways and take on various alternative forms, specific embodiments thereof are shown in the drawings and described in detail below as examples.

Like reference numerals refer to like elements in principle throughout the specification. Further, elements with the same function within the scope of the same idea shown in the drawings of each embodiment will be described using the same reference numerals, and the descriptions thereof will not be repeated.

When it is determined that detailed descriptions of related well-known functions or configurations may unnecessarily obscure the gist of the present invention, detailed descriptions thereof will be omitted. Further, the ordinal numbers (for example, first, second, etc.) used in description of the specification are used only to distinguish one element from another element.

Further, the term "module," "unit," "part," or "portion" of an element used herein is assigned or incorporated for convenience of specification description, and the term itself does not have a distinct meaning or role.

As used herein, the singular forms "a" and "an" are intended to include the plural forms as well, unless the context clearly indicates otherwise.

It will be further understood that the terms "comprise," "comprising," "include," and/or "including" used herein specify the presence of stated features or elements, but do not preclude the presence or addition of one or more other features or elements.

Sizes of elements in the drawings may be exaggerated for convenience of explanation. In other words, since sizes and thicknesses of elements in the drawings are arbitrarily illustrated for convenience of explanation, the following embodiments are not limited thereto.

When a certain embodiment may be implemented differently, a specific process order may be performed differently from the described order. For example, two consecutively described processes may be performed substantially at the same time or performed in an order opposite to the described order.

In the following embodiments, when a first element is referred to as being "connected" to a second element, it includes not only a case where the two elements are "directly connected," but also a case where the two elements are "indirectly connected" with a third element interposed therebetween. For example, in this specification, when a first element is referred to as being "electrically connected" to a second element, it includes not only a case where the two elements are "directly electrically connected," but also a case where the two elements are "indirectly electrically connected" with a third element interposed therebetween.

The kit for quantitative analysis of Glycated Albumin (GA) according to an embodiment of the present application may comprises: a first composition including a reaction buffer for inducing binding with albumin present in a blood sample; a second composition including a decomposition reagent for a decomposition reaction to break down glycated albumin (GA) included in the blood sample into glycated amino acids; a third composition including a reaction reagent for an enzymatic reaction of the glycated amino acids present in the blood sample; and a fourth composition including a chromogenic agent being oxidized by hydrogen peroxide (H₂O₂) generated from the enzymatic reaction of the glycated amino acids, wherein the GA quantitative analysis kit may include at least one reagent fixing part, and the second composition to the fourth composition may be each independently fixed to the reagent fixing part.

According to an embodiment of the present application, wherein the first composition may be selected from the group consisting of Bromocresol Purple (BCP) and Bromocresol Green (BCG).

According to an embodiment of the present application, wherein the reaction buffer may be selected from the group consisting of HEPES, PB, Tris-HCl, Tris-Base, and PBS.

According to an embodiment of the present application, wherein the reaction buffer may be selected from the group consisting of Tris-HCl and Tris-Base, and the pH of the first composition may be in the range of 4.1 to 10.0.

According to an embodiment of the present application, wherein the reaction buffer may be Tris-HCl, and the decomposition reaction of glycated albumin and the enzymatic reaction of glycated amino acids may be performed under a slightly alkaline pH condition.

According to an embodiment of the present application, wherein the first composition may further include a surfactant selected from the group consisting of CHAPSO, Triton X-100, and Tween 20.

According to an embodiment of the present application, wherein the first composition may further include Triton X-100 surfactant at a concentration of more than 0% and up to 0.1%.

According to an embodiment of the present application, wherein the reaction buffer may include Tris-HCl, the first composition may further include Triton X-100 surfactant at a concentration of more than 0% and up to 0.1%, and the decomposition reaction of glycated albumin and the enzymatic reaction of glycated amino acids may be performed under a slightly alkaline pH condition.

According to an embodiment of the present application, wherein the decomposition reagent may be proteinase.

According to an embodiment of the present application, wherein the reaction reagent may include ketoamine oxidase (KAO) and peroxidase (POD).

According to an embodiment of the present application, wherein the chromogenic agent may be DA-67.

According to an embodiment of the present application, wherein the GA quantitative analysis kit may further include a fifth composition comprising an oxidase to prevent interference from ascorbic acid present in the blood sample, and the oxidase may include ascorbate oxidase (ASOx).

According to an embodiment of the present application, wherein the second composition may further include a stabilizer for immobilizing the decomposition reagent onto the at least one reagent fixing part in solid form, and the stabilizer may include disaccharides, DEAE-Dextran, and NPS.

According to an embodiment of the present application, wherein the third composition may further include a stabilizer for immobilizing the reaction reagent onto the at least one reagent fixing part in solid form, and the stabilizer may include disaccharides, DEAE-Dextran, and NPS.

According to an embodiment of the present application, wherein the fourth composition may further include a stabilizer for immobilizing the chromogenic agent onto the at least one reagent fixing part in solid form, and the stabilizer may include disaccharides.

According to an embodiment of the present application, wherein the fifth composition may further include a stabilizer for immobilizing ascorbate oxidase onto the at least one reagent fixing part in solid form, and the stabilizer may include disaccharides, DEAE-Dextran, and NPS.

According to an embodiment of the present application, wherein the third composition may be immobilized in solid state onto a first reagent fixing part located in the first region of the GA quantitative analysis kit, and the second composition may immobilized in solid state onto a second reagent fixing part located in the second region, which is partitioned from the first region, of the GA quantitative analysis kit.

According to an embodiment of the present application, wherein the GA quantitative analysis kit may be configured such that the reaction buffer is mixed with the reaction reagent fixed to the first reagent fixing part before being mixed with the decomposition reagent fixed to the second reagent fixing part, and the reaction reagent may be characterized by performing the enzymatic reaction with the glycated amino acids from the point when the glycated amino acids are generated by the decomposition reaction caused by the decomposition reagent.

According to an embodiment of the present application, wherein the GA quantitative analysis kit may comprise a body including an upper plate and a lower plate configured in a facing arrangement, the third composition may be fixed to the first reagent fixing part located in the first region of the upper plate, and the fifth composition may be fixed to the third reagent fixing part located in the first region of the lower plate, with the first reagent fixing part and the third reagent fixing part configured to face each other.

According to an embodiment of the present application, wherein the second composition may be fixed to the second reagent fixing part located in the second region of the upper plate, and the fourth composition may be fixed to the fourth reagent fixing part located in the second region of the lower plate, with the second reagent fixing part and the fourth reagent fixing part configured to face each other.

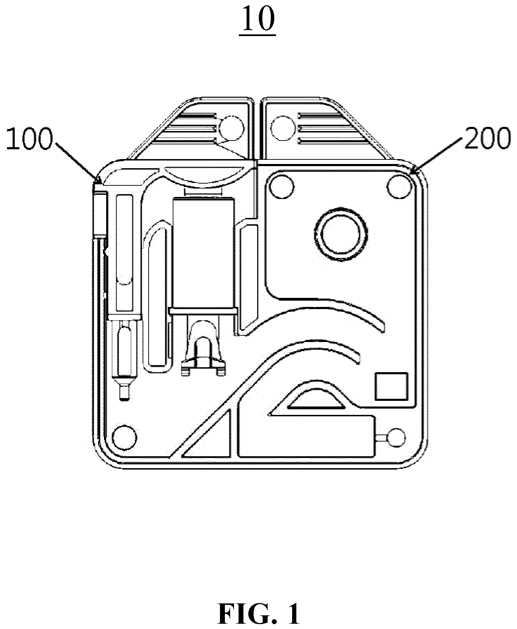

Hereinafter, with reference to Figures 1 to 17, the structure of the kit for quantitative analysis of Glycated Albumin (GA), the method for quantitative analysis of GA, and/or the apparatus for quantitative analysis of GA according to an embodiment of the present application will be described in greater detail.

Fig. 1 is a schematic diagram of the glycated albumin quantitative analysis kit according to an embodiment of the present application.

The kit for quantitative analysis of Glycated Albumin (hereinafter referred to as "GA") 10 according to an embodiment of the present application may include a sample collector 100 for supplying a biological sample and/or a main cartridge 200 into which the sample collector 100 can be inserted and received.

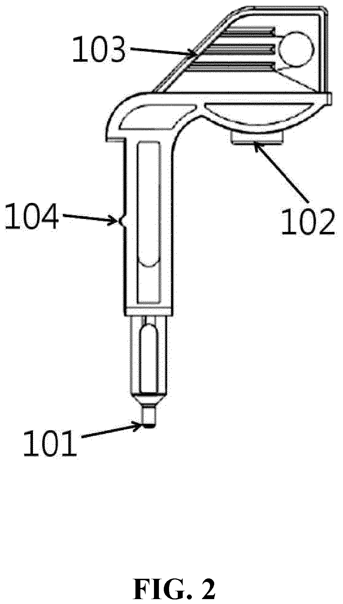

Fig. 2 is a schematic diagram of the sample collector 100 of the glycated albumin quantitative analysis kit according to an embodiment of the present application.

The sample collector 100 according to an embodiment of the present application may be configured to collect a predetermined amount of a biological sample (e.g., a blood sample) intended for analysis. Specifically, the sample collector 100 may include a capillary-type sample inlet 101 and may collect the biological sample (e.g., a blood sample) to be analyzed through the capillary provided in the sample inlet 101, injecting the collected biological sample into the main cartridge 200.

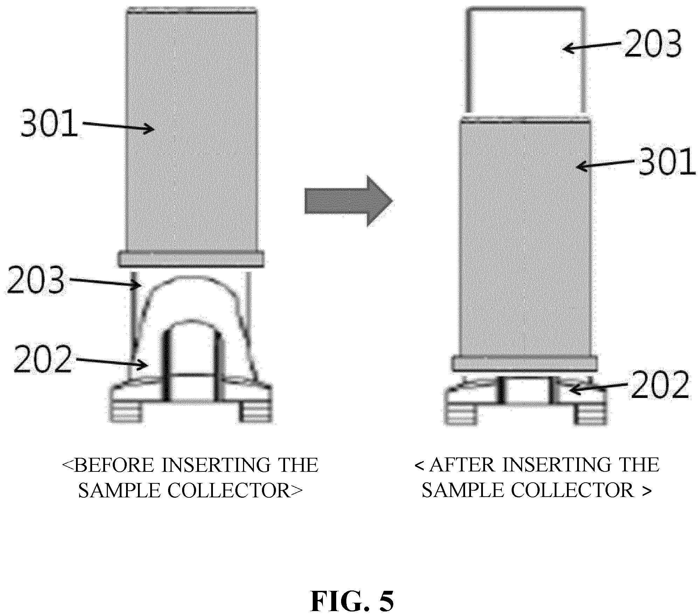

Furthermore, the sample collector 100 may include a protrusion 102 designed to contact a solution cell 301 inside the main cartridge 200 and push it inward when the sample collector 100 is inserted into the receiving part 201 of the main cartridge 200, described later. Specifically, the protrusion 102 may be positioned on the sample collector 100 to contact the solution cell 301 when the sample collector 100 is inserted into the receiving part 201 of the main cartridge 200. Consequently, as the sample collector 100 is inserted into the receiving part 201 of the main cartridge 200, the solution cell 301 may be moved inward toward the interior of the main cartridge 200, causing the cover tape 302 of the solution cell 301 to be removed or ruptured. This will be described in more detail with reference to Fig. 5.

Additionally, the sample collector 100 may further include a handle 103 and/or a locking clip 104. Specifically, the handle 103 is a component designed to facilitate the transportation or use of the sample collector 100 and is not limited to the structure shown in Fig. 2. The locking clip 104 may be provided on one side of the sample collector 100 to secure the sample collector 100 to the main cartridge 200 when inserted into main cartridge 200. The locking clip 104 is designed to match the size and shape of a locking groove 211 provided in the receiving part 201 of the main cartridge 200. The locking clip 104 of the sample collector 100 and the locking groove 211 of the main cartridge 200 can engage with each other. As a result, the inserted sample collector 100 may be fixed to the main cartridge 200, preventing movement or detachment of the sample collector 100 even if the GA quantitative analysis kit rotates during analysis.

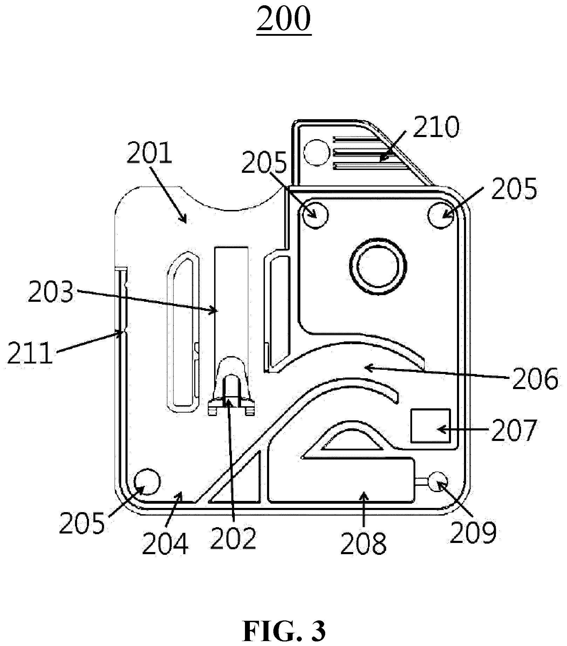

Fig. 3 is a schematic diagram of the main cartridge 200 of the glycated albumin quantitative analysis kit according to an embodiment of the present application.

The main cartridge 200 according to an embodiment of the present application may include a receiving part 201 into which the sample collector 100 can be inserted. Furthermore, the receiving part 201 may include a locking groove 211 configured to engage with the locking clip 104 of the sample collector 100, as described above, to secure the sample collector 100.

The main cartridge 200 may also include a moving frame 203 configured to fix and move the solution cell 301 provided inside the main cartridge. Specifically, the moving frame 203 may be configured to secure the solution cell 301 prior to the insertion of the sample collector 100. When the sample collector 100 is inserted, the solution cell 301, pushed by the protrusion 102 of the sample collector 100, may move along a moving path in the moving frame 203.

The main cartridge 200 may further include a cover tape rupturing part 202 configured to remove or rupture the cover tape 302 of the solution cell 301 as the solution cell 301 moves along the moving frame 203. This will be described in greater detail with reference to Fig. 5.

The main cartridge 200 may include a mixing part 204 (or mixing region) where the biological sample (e.g., a blood sample) discharged from the sample collector 100 through the receiving part 201 mixes with the composition (hereinafter referred to as the first composition) discharged from the solution cell 301.

The main cartridge 200 may also include at least one reagent fixing part 205 (referred to as a sample fixing part) introduced with chemical reagents that can react with the first composition and biological sample mixed in the mixing part 204 to induce enzymatic reaction and/or antigen-antibody reactions.

The main cartridge 200 may further include a flow path 206 through which the mixed first composition and biological sample in the mixing part 204 can move. Specifically, the analysis sample may travel, through the flow path 206, between the mixing part 204, the reagent fixing part 205, and/or a measurement unit 207 that optically measures the analysis sample. The structure of the flow path 206 is not limited, provided it is designed to allow the analysis sample to move by gravity when the main cartridge 200 is tilted.

The main cartridge 200 according to an embodiment of the present application may include a measurement unit 207 for measuring the reaction results performed at the reagent fixing part 205. The GA quantitative analysis apparatus according to an embodiment of the present application can quantify the analysis sample (e.g., GA) through optical analysis, such as UV/VIS, via the measurement unit 207.

The main cartridge 200 may include a waste solution treatment part 208 for collecting waste solutions which analyse was completed via the measurement unit 207. The waste solution treatment part 208 allows for the collection and separate disposal of waste solutions, which are a type of medical waste. Collection of waste solutions via the waste solution treatment part 208 can be achieved by absorbing the waste solution into highly absorbent cotton, absorbent filters, or polymer-based absorbent materials, placed in the waste solution treatment part 208.

The main cartridge 200 may further include an air vent 209 to facilitate the smooth movement and absorption of the waste solution into the absorbent materials. Through the air vent 209, the transfer of waste solutions to the waste solution treatment part 208 and their subsequent collection can be more efficiently performed.

The main cartridge 200 may also include a handle 210 to facilitate its transportation and use. The structure of the handle 210 is not limited to the form shown in Fig. 3.

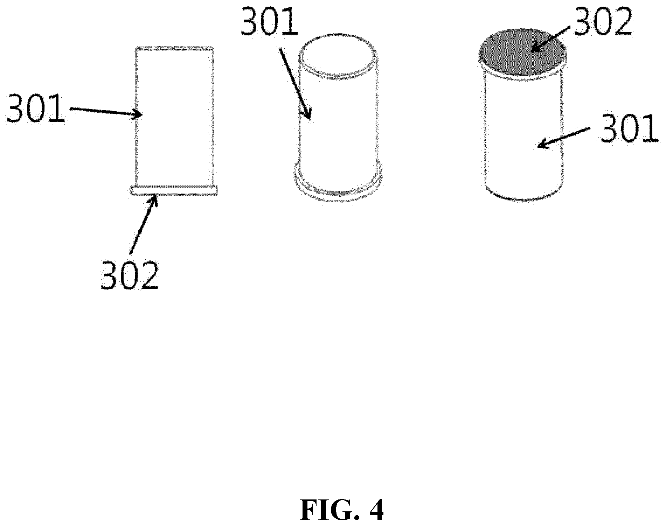

Hereinafter, with reference to Figures 4 and 5, a more detailed description will be provided of the aspects in which the first composition of the solution cell 301 flows into the mixing part 204 of the main cartridge 200 when the sample collector 100 is inserted into the receiving part 201 of the main cartridge 200. Fig. 4 is a schematic diagram of the solution cell 301 of the GA quantitative analysis kit according to an embodiment of the present application. Fig. 5 is a diagram illustrating the aspects in which the composition stored in the solution cell 301 flows into the mixing region of the main cartridge 200 as the sample collector 100 is inserted into the main cartridge 200, according to an embodiment of the present application.

The main cartridge 200 according to an embodiment of the present application may include a solution cell 301 that stores a first composition for reacting with the biological sample from the sample collector 100. The solution cell 301 includes an opening at one end, sealed with a cover tape 302, to prevent the first composition stored inside from leaking. The opening sealed with the cover tape 302 of solution cell 301 is positioned to face the cover tape rupturing part 202 of the main cartridge 200.

Before the sample collector 100 is inserted into the receiving part 201 of the main cartridge 200, the opening of the solution cell 301 sealed with the cover tape 302 may be positioned apart from the cover tape rupturing part 202. Before the sample collector 100 is inserted into the receiving part 201 of the main cartridge 200, the opening of the solution cell 301 sealed with the cover tape 302 may be positioned apart from the cover tape rupturing part 202. When the sample collector 100 is inserted into the receiving part 201 of the main cartridge 200, the protrusion 102 of the sample collector 100 applies pressure to the opposite end of the solution cell 301, away from the opening sealed with the cover tape 302. This applied pressure causes the solution cell 301 to move along the moving path of the moving frame 203.

As a result, when the cover tape 302 of the solution cell 301 comes into contact with the cover tape rupturing part 202, the cover tape 302 of the solution cell 301 is removed or ruptured. The first composition stored in the solution cell 301 is then transferred through a flow path or hollow structure formed in the cover tape rupturing part 202 into the mixing part 204 of the main cartridge 200. In the mixing part 204, the reaction buffer mixes with the biological sample (e.g., a blood sample) supplied from the sample collector 100.

Specifically, the first composition flowing into the mixing part 204 may be designed to contact the sample inlet 101 of the sample collector 100. This contact allows the biological sample contained in the capillary-type sample inlet 101 to move through the sample inlet 101 into the mixing part 204 of the main cartridge 200. Consequently, in the mixing part 204 of the main cartridge 200, the first composition stored in the solution cell 301 and the biological sample collected by the sample collector 100 can be mixed.

In Figures 4 and 5, the shape of the solution cell 301 is illustrated as a specific example. However, this is merely an example, and the shape of the solution cell 301 is not limited as long as it provides a structure suitable for storing the first composition. Similarly, the material of the cover tape 302 is not particularly limited as long as it prevents the first composition from leaking while allowing for easy removal or rupture.

Additionally, Figure 5 illustrates the cover tape rupturing part 202 in a specific shape for explanation purposes. However, this is also merely an example, and the shape of the cover tape rupturing part 202 is not limited, provided it facilitates the removal or rupture of the cover tape. Suitable structures include, but are not limited to, needle-shaped or edge-shaped configurations.

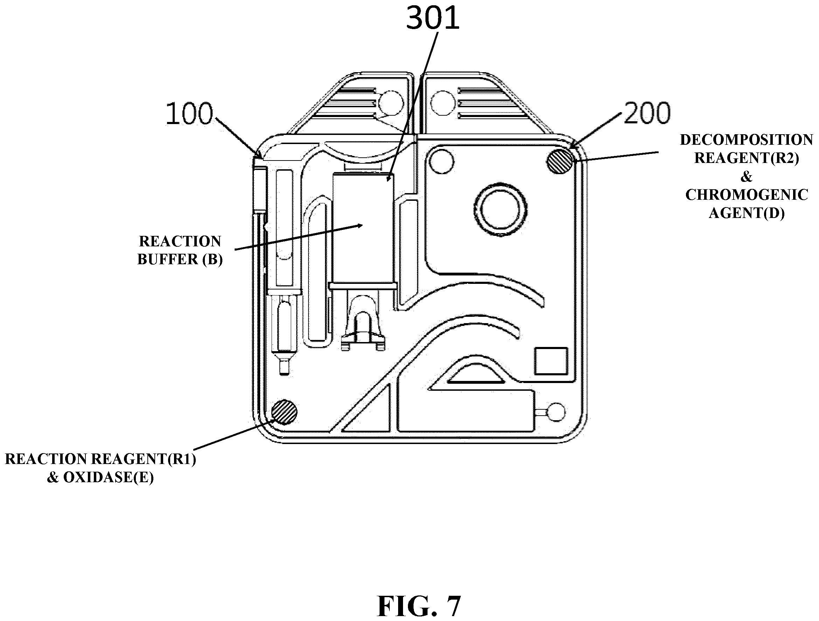

The GA quantitative analysis kit 10 according to an embodiment of the present application may be used to quantitatively analyze GA present in blood (e.g., plasma, serum). According to one embodiment, the GA quantitative analysis kit 10 may be configured to quantitatively analyze GA using a BCP method and/or Enzymatic method. Hereinafter, with reference to Figures 7 to 9, a more detailed description will be provided of the aspects of quantitatively analyzing GA using the GA quantitative analysis kit 10 according to an embodiment of the present application.

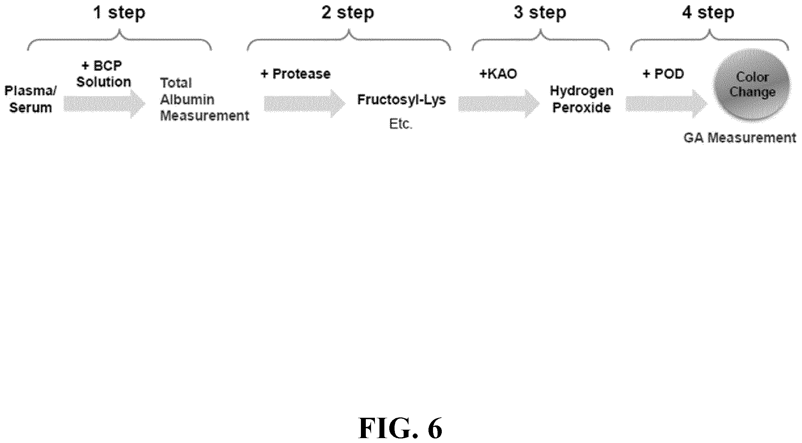

The analysis method for quantifying GA can be broadly divided into four steps, as shown in FIG. 6 below. FIG. 6 illustrates one aspect of an analysis method for quantifying GA according to one embodiment of the present application.

The first step involves quantifying the total amount (or concentration) of albumin present in the blood. According to one embodiment, the total amount of albumin in the blood (plasma or serum) can be quantified using the Bromocresol Purple (BCP) method. Specifically, BCP binds to albumin to form a BCP complex. By measuring the absorbance of the sample containing the BCP complex, the total amount of albumin can be quantified.

The second step involves decomposing albumin with a tertiary structure (including glycated albumin). Specifically, the tertiary structure of albumin can be decomposed into at least one glycated amino acid (e.g., Fructosyl-Lys) using a decomposition enzyme, such as protease or proteinase.

The third step involves generating hydrogen peroxide through an enzymatic reaction of glycated amino acids. According to one embodiment, glycated amino acids can be decomposed into amino acids and glucose by a reaction enzyme (e.g., Ketoamine Oxidase (KAO)), during which hydrogen peroxide is generated.

The fourth step involves oxidizing a chromogenic agent (e.g., DA-67) in the presence of a reaction enzyme (e.g., Peroxidase (POD)) using the generated hydrogen peroxide. As the chromogenic agent undergoes oxidation, a color change occurs. By measuring the absorbance at a specific wavelength corresponding to this color change, the amount of glycated albumin can be quantified.

However, the aforementioned analysis mechanism for quantifying GA is merely an example, and any suitable GA quantitative analysis method may be employed. Depending on the GA quantitative analysis method, the GA quantitative analysis kit according to an embodiment of the present application can be appropriately modified.

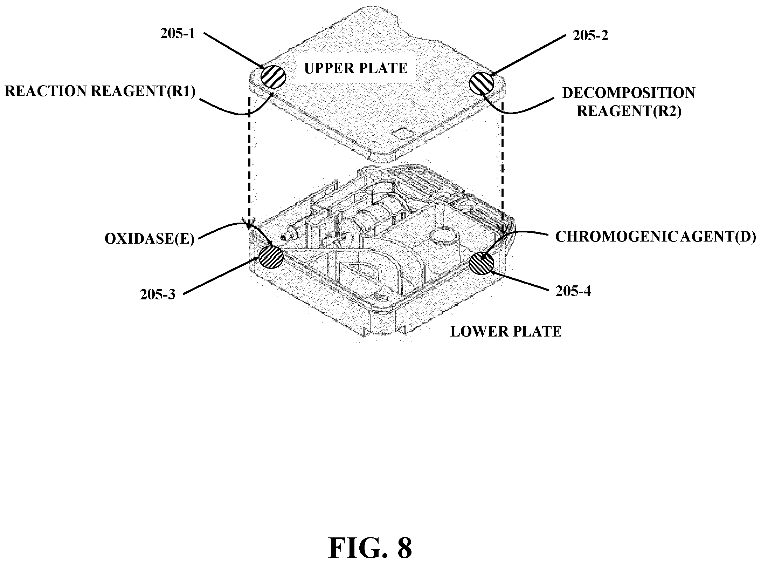

Figs. 7 and 8 are diagrams showing the specific structure of the GA quantitative analysis kit 10 according to an embodiment of the present application.

The solution cell 301 of the GA quantitative analysis kit 10 according to an embodiment of the present application may store a composition (hereinafter referred to as the first composition) containing a reagent for inducing binding with albumin present in a blood sample. According to one embodiment, the first composition may include Bromocresol Purple (BCP) for forming a complex by binding with albumin present in the blood sample and a reaction buffer (B) for establishing the reaction environment. Bromocresol Purple (BCP) is exemplified as a substance for forming a complex by binding with albumin, but it may be replaced with any suitable substance having similar characteristics to BCP, including Bromocresol Green.

The reaction buffer B according to one embodiment may be selected from the group consisting of HEPES, PB, Tris-HCl, Tris-Base, and PBS. In this case, the pH of the first composition containing the reaction buffer B can be implemented within a pH range of 4.1 to 10.0. More preferably, the pH of the first composition can be implemented within a pH range of 5.8 to 8.0.

The reaction buffer B according to one embodiment may be selected from the group consisting of Tris-HCl and Tris-Base. In this case, the pH of the first composition containing the reaction buffer B can be implemented within a pH range of 4.1 to 10.0. More preferably, the pH of the first composition can be implemented within a pH range of 5.8 to 8.0.

The reaction buffer B according to one embodiment may be Tris-HCl. In this case, the pH of the first composition containing the reaction buffer B can be implemented within a pH range of 4.1 to 10.0. More preferably, the pH of the first composition can be implemented within a pH range of 4.1 to 8.0. Preferably, the pH of the first composition can be implemented within a pH range of 5.8 to 8.0.

More preferably, the analysis environment established by the reaction buffer B (e.g., the analysis environment for the decomposition reaction of glycated albumin and the enzymatic reaction of glycated amino acids, described later) may be maintained in a slightly alkaline state (e.g., pH 8). Specifically, the decomposition reaction of glycated albumin and the enzymatic reaction of glycated amino acids, as described later, may be performed under a slightly alkaline condition (e.g., pH 8).

According to the glycated albumin quantitative analysis kit, the glycated albumin quantitative analysis method, and/or the device for performing the method according to an embodiment of the present application, maintaining the pH of the analysis environment at a slightly alkaline level, which is the optimal reaction environment for the enzyme Ketoamine Oxidase (KAO), can provide the effect of improving the accuracy of glycated albumin quantification by the enzymatic method.

The first composition according to one embodiment may further include a surfactant to create an optimal analysis environment. More specifically, the first composition may further include a surfactant to prevent the precipitation of proteins.

The surfactant according to one embodiment may be selected from the group consisting of CHAPSO, Triton X-100, and Tween 20. Preferably, the surfactant may be Triton X-100, and the concentration of Triton X-100 may be included in the first composition at a concentration greater than 0% and up to 0.1%.

According to the glycated albumin quantitative analysis kit, the glycated albumin quantitative analysis method, and/or the device for performing the method according to an embodiment of the present application, the combination of an optimal reaction buffer and a surfactant may provide the effect of implementing the BCP reaction for quantifying the total amount of albumin and the enzymatic reaction for quantifying the amount of glycated albumin on a single kit. Furthermore, this enables the miniaturization of the glycated albumin quantitative analysis kit. Additionally, it may provide the effect of reducing the analysis time for glycated albumin and enhancing user convenience.

According to an embodiment of the present application, the GA quantitative analysis kit 10 may include a composition (hereinafter referred to as the second composition) containing a decomposition reagent (R2) for a decomposition reaction to break down glycated albumin (GA) included in a blood sample into glycated amino acids. The decomposition reagent (R2) may be a proteinase. The decomposition reagent (R2) performs the function of breaking down the tertiary protein structure of glycated albumin, converting it into glycated amino acids, which are in a form capable of participating in the enzymatic reaction described later.

According to an embodiment of the present application, the GA quantitative analysis kit 10 may include a composition (hereinafter referred to as the third composition) containing a reaction reagent (R1) for the enzymatic reaction of glycated amino acids included in a blood sample. The reaction reagent (R1) may include Ketoamine Oxidase (KAO) and/or Peroxidase (POD). The reaction reagent (R1) performs the function of carrying out an enzymatic reaction with the glycated amino acids generated by the decomposition reagent (R2) and oxidizing the result of the enzymatic reaction. Specifically, the Ketoamine Oxidase (KAO) in the reaction reagent (R1) functions to generate hydrogen peroxide through an enzymatic reaction with glycated amino acids, while the Peroxidase (POD) reacts with the hydrogen peroxide to oxidize the chromogenic agent, which will be described later.

According to an embodiment of the present application, the GA quantitative analysis kit 10 may include a composition (hereinafter referred to as the fourth composition) containing a chromogenic agent that is oxidized by the hydrogen peroxide generated from the enzymatic reaction of glycated amino acids. The chromogenic agent may be DA-67.

According to an embodiment of the present application, the GA quantitative analysis kit 10 may include a composition (hereinafter referred to as the fifth composition) containing an oxidase (E) for preventing interference from ascorbic acid present in the blood sample. The oxidase (E) may include ascorbate oxidase (ASOx).

According to an embodiment of the present application, the GA quantitative analysis kit 10 may further include a stabilizer for immobilizing the second composition to fifth composition in a solid state on at least one reagent fixing part 205.

According to one embodiment, the second composition may further include a stabilizer for immobilizing the decomposition reagent (R2) in a solid state, wherein the stabilizer may be selected from the group consisting of disaccharides (e.g., trehalose, sucrose), DEAE-Dextran, and NPS. More preferably, the stabilizer for the second composition may include trehalose, DEAE-Dextran, and NPS.

According to one embodiment, the third composition may further include a stabilizer for immobilizing the reaction reagent (R1) in a solid state, wherein the stabilizer may be selected from the group consisting of disaccharides (e.g., trehalose, sucrose), DEAE-Dextran, and NPS. More preferably, the stabilizer for the third composition may include trehalose, DEAE-Dextran, and NPS.

According to one embodiment, the fourth composition may further include a stabilizer for immobilizing the chromogenic agent (D) in a solid state, wherein the stabilizer may be a disaccharide (e.g., trehalose, sucrose). More preferably, the stabilizer for the fourth composition may be trehalose.

According to one embodiment, the fifth composition may further include a stabilizer for immobilizing the oxidase (E) in a solid state, wherein the stabilizer may be selected from the group consisting of disaccharides (e.g., trehalose, sucrose), DEAE-Dextran, and NPS. More preferably, the stabilizer for the fifth composition may include trehalose, DEAE-Dextran, and NPS.

As described above, the GA quantitative analysis kit 10 may include at least one reagent fixing part 205. According to one embodiment, the second composition to fifth composition may each independently be fixed to at least one reagent fixing part 205.

According to one embodiment, the third composition containing the reaction reagent (R1) may be fixed to the first reagent fixing part 205-1 located in the first region of the GA quantitative analysis kit 10. The first region may be an area positioned on the mixing part 204. In contrast, the second composition containing the decomposition reagent (R2) may be fixed to the second reagent fixing part 205-2 located in the second region of the GA quantitative analysis kit 10. The second region is a separate area partitioned from the first region. The first and second regions may be connected via a flow path 206, and the GA quantitative analysis kit 10 may be configured such that the measurement unit 207 is positioned midway along the path through which the blood sample moves from the first region to the second region.

Referring to Fig. 8, the GA quantitative analysis kit 10 may include a body comprising an upper plate and a lower plate configured in a facing arrangement.

According to one embodiment, the third composition containing the reaction reagent (R1) may be dispensed and dried on the first reagent fixing part 205-1 provided in the first region of the upper plate of the body. In contrast, the fifth composition containing the oxidase (E) may be dispensed and dried on the third reagent fixing part 205-3 provided in the first region of the lower plate of the body. Furthermore, the first reagent fixing part 205-1 provided on the upper plate and the third reagent fixing part 205-3 provided on the lower plate of the body may be configured in a facing arrangement. In Fig. 8, it is described that the third composition containing the reaction reagent (R1) is provided on the upper plate of the body, while the fifth composition containing the oxidase (E) is provided on the lower plate. However, this is merely an example, and the compositions can be provided on the multiple reagent fixing parts 205 in any appropriate configuration, such as the third composition containing the reaction reagent (R1) being provided on the lower plate and the fifth composition containing the oxidase (E) being provided on the upper plate.

According to one embodiment, the second composition containing the decomposition reagent (R2) may be dispensed and dried on the second reagent fixing part 205-2 provided in the second region of the upper plate of the body. In contrast, the fourth composition containing the chromogenic agent (D) may be dispensed and dried on the fourth reagent fixing part 205-4 provided in the second region of the lower plate of the body. Furthermore, the second reagent fixing part 205-2 provided on the upper plate and the fourth reagent fixing part 205-4 provided on the lower plate of the body may be configured in a facing arrangement. In Fig. 8, it is described that the second composition containing the decomposition reagent (R2) is provided on the upper plate of the body, while the fourth composition containing the chromogenic agent (D) is provided on the lower plate. However, this is merely an example, and the compositions can be provided on the multiple reagent fixing parts 205 in any appropriate configuration, such as the second composition containing the decomposition reagent (R2) being provided on the lower plate of the body and the fourth composition containing the chromogenic agent (D) being provided on the upper plate of the body.

According to an embodiment of the present application, the third composition containing the reaction reagent (R1) is fixed to the first reagent fixing part 205-1 provided on the mixing part 204, and the second composition containing the decomposition reagent (R2) is fixed to the second reagent fixing part 205-2 partitioned from the mixing part 204. This configuration ensures that the reaction buffer released from the solution cell 301 (and/or the blood sample released from the sample collector 100) is mixed with the reaction reagent (R1) fixed to the first reagent fixing part 205-1 before coming into contact with the decomposition reagent (R2) fixed to the second reagent fixing part 205-2. However, the reaction reagent (R1) does not perform the enzymatic reaction from the point when the reaction buffer and the blood sample are mixed. Instead, it is characterized by performing the enzymatic reaction with glycated amino acids from the point when glycated amino acids are generated through the decomposition reaction by the decomposition reagent (R2), that is, from the point when the glycated albumin included in the analytical sample is decomposed by the decomposition reagent (R2) of the second reagent fixing part (205-2) to generate glycated amino acids. According to the glycated albumin quantitative analysis kit, the glycated albumin quantitative analysis method, and/or the device for performing the method as described in an embodiment of the present application, fixing the second composition containing the decomposition reagent (R2) to the second reagent fixing part 205-2 instead of the first reagent fixing part 205-1 prevents the loss of the BCP binding site present in albumin, thereby improving the accuracy of total albumin quantification using the BCP analysis method.

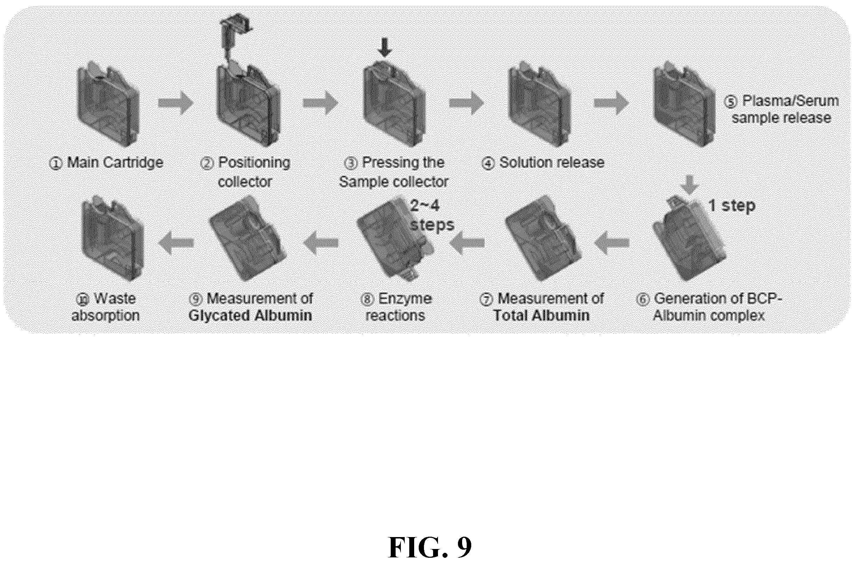

Fig. 9 is a diagram illustrating the analysis aspect for quantifying glycated albumin in a blood sample according to an embodiment of the present application.

Referring to Fig. 9, the glycated albumin quantitative analysis method according to an embodiment of the present application may be implemented as follows.
1) Prepare the main cartridge 200.
2) Position the sample collector 100 on the receiving part 201 of the main cartridge 200.
3) Apply pressure to the sample collector 100 in the direction toward the receiving part 201 of the main cartridge 200.
4) As the protrusion 102 of the sample collector 100 applies pressure to the solution cell 301, the solution cell 301 moves, as described above, through the moving frame 203 in the direction of the cover tape rupturing part 202. The cover tape rupturing part 202 comes into contact with the cover tape 302 of the solution cell 301, causing the solution (the first composition) stored inside the solution cell 301 to flow into the mixing part 204 of the main cartridge 200.
5) As the first composition flows into the mixing part 204, it comes into contact with the blood sample from the sample collector 100, causing the blood sample (e.g., plasma, serum) to also flow into the mixing part 204 of the main cartridge 200.
6) At this stage, on the mixing part 204, the first composition containing the reaction buffer (B) and Bromocresol Purple (BCP) is mixed with the blood sample. In this process, albumin (meaning including both non-glycated albumin and glycated albumin) in the blood sample binds with BCP, forming a BCP complex.

Meanwhile, on the mixing part 204, the reaction reagent (R1) and the oxidase (E), which are fixed to the reagent fixing parts 205-1 and 205-2 provided on the mixing part 204, also mix with the blood sample. However, the reaction reagent (R1) does not react with albumin or glycated albumin having a tertiary structure. Only when the blood sample reaches the reagent fixing parts 205-3 and 205-4, where the decomposition reagent (R2) is fixed, and the tertiary structure of albumin and/or glycated albumin is decomposed by the decomposition reagent (R2) to generate glycated amino acids, does the enzymatic reaction proceed.

Meanwhile, according to an embodiment of the present application, the GA quantitative analysis kit 10 rotates by a predetermined angle due to an external force (e.g., rotational force applied by a quantitative analysis device, as described later). Consequently, the analytical sample including blood sample inside the main cartridge 200 moves through the flow path 206 of the main cartridge 200 under the influence of gravity.

7) By an external force applied to the GA quantitative analysis kit 10, the blood sample moves to the measurement unit 207. While the sample is positioned on the measurement unit 207, the absorbance corresponding to the total amount (or concentration) of albumin (Total Albumin) in the blood sample (hereinafter referred to as the first absorbance) can be measured.

Meanwhile, as described above, the GA quantitative analysis kit 10 may include a measurement unit 207 for quantifying GA included in the blood sample. According to one embodiment, the GA quantitative analysis kit 10 may be configured such that the blood sample passes through the measurement unit 207 while moving from the mixing part 204 through the flow path 206 to the reagent fixing parts 205-3 and 205-4, where the enzymatic reaction is performed. In this embodiment, the absorbance before the enzymatic reaction, corresponding to the concentration of total albumin, and the absorbance after the enzymatic reaction, corresponding to the concentration of glycated albumin, can both be easily measured. This may provide the effect of enabling the glycated albumin ratio to be quantified more conveniently.

8) By an external force applied to the GA quantitative analysis kit 10, the analyte sample containing the blood sample moves from the measurement unit 207 to the reagent fixing parts 205-3 and/or 205-4, where the decomposition reagent (R2, e.g., Protease) is fixed. On the reagent fixing parts 205-3 and/or 205-4, the glycated albumin included in the blood sample is decomposed into glycated amino acids by the decomposition reagent (R2).

Furthermore, an enzymatic reaction (as shown in Fig. 9, "Enzyme Reaction") occurs between the decomposed glycated amino acids and the reaction reagent (R1, e.g., KAO) that had been mixed earlier at the reagent fixing parts 205-1 and/or 205-2. Through this enzymatic reaction, hydrogen peroxide is generated, and as the generated hydrogen peroxide is oxidized by the reaction reagent (R1, e.g., POD), electrons are released, causing the chromogenic agent (D) to change color, resulting in a change in absorbance.

9) By an external force applied to the GA quantitative analysis kit 10, the sample after the enzymatic reaction moves from the reagent fixing parts 205-3 and/or 205-4 to the measurement unit 207. While the sample is positioned on the measurement unit 207, the measurement value related to the absorbance of the sample after the enzymatic reaction (the second absorbance) can be measured. The second absorbance may correspond to the amount (or concentration) of glycated albumin present in the blood sample.

Furthermore, based on the first absorbance related to the total albumin concentration measured before the enzymatic reaction at the measurement unit 207 and the second absorbance related to the glycated albumin concentration measured after the enzymatic reaction at the measurement unit 207, the ratio of glycated albumin to total albumin in the blood sample can be quantified.

10) The waste from the completed quantitative analysis, moves from the measurement unit 207 to the waste solution treatment part 208 due to the external force applied to the GA quantitative analysis kit 10, where it is collected.

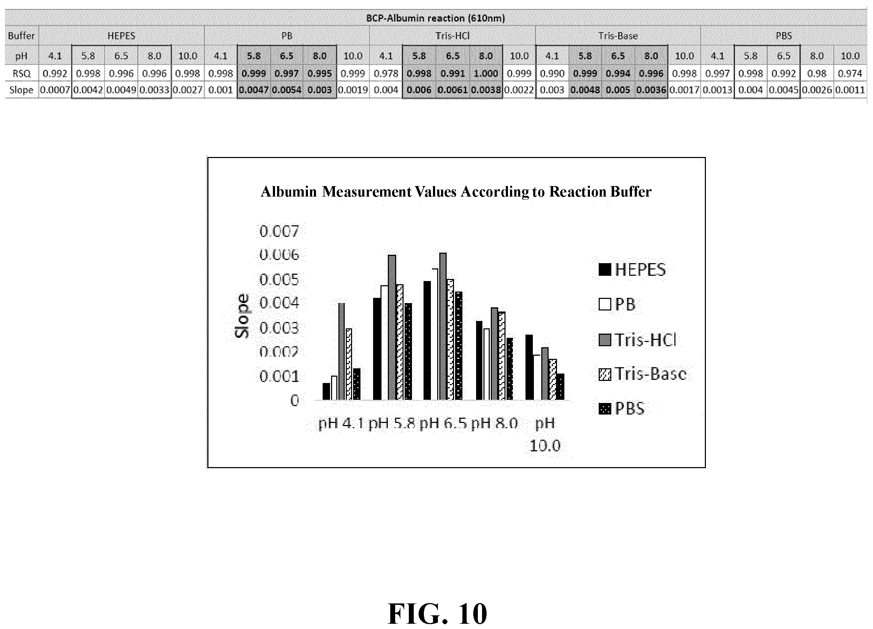

Hereinafter, with reference to FIGS. 10 to 16, the present invention will be described in detail through experimental examples. However, the following experimental examples are merely illustrative and should not be construed as limiting.

### <Experimental Example 1: Evaluation of Albumin Measurement Performance Based on Reaction Buffers>

### 1. Experimental Method

To determine the optimal reaction buffer and reaction conditions(pH) for albumin analysis, five candidate buffers (HEPES, Phosphate Buffer, Tris-HCl, Tris-Base, and Phosphate-buffered Saline) were selected. For each candidate buffer, five pH conditions (pH 4.1, 5.8, 6.5, 8.0, and 10.0) were tested, resulting in a total of 25 buffer and pH combinations. The measurement performance of albumin-BCP analysis was evaluated for all combinations. Specifically, the candidate buffers were supplemented with an appropriate concentration of Bromocresol Purple (BCP) reagent and the non-ionic surfactant Triton X-100.

The test samples were prepared using albumin powder (Albumin from Human Serum, lyophilized powder, Sigma Aldrich; A3782). Specifically, a low-concentration sample (16.6 g/L) was prepared by dissolving 83 mg of albumin powder in 5 mL of deionized water (DIW), and a high-concentration sample (64.8 g/L) was prepared by dissolving 130 mg of albumin powder in 5 mL of DIW. A medium-concentration sample (40.4 g/L) was prepared by mixing the low-concentration sample and high-concentration sample at a 1:1 ratio.

Furthermore, a mid-low concentration sample (28.4 g/L) was prepared by mixing the low-concentration sample and the medium-concentration sample at a 1:1 ratio, and a mid-high concentration sample (52.6 g/L) was prepared by mixing the medium-concentration sample and the high-concentration sample at a 1:1 ratio, resulting in a total of five sample concentrations. The prepared samples were verified for albumin content (g/L) using a reference reagent (Lucica GA-L, Asahi Kasei Pharma) and an automatic biochemical analyzer (RX Imola, RANDOX). Each candidate buffer was applied to the albumin samples (Sigma Aldrich) under five different pH conditions. Furthermore, the 25 candidate buffers and five test samples were mixed in a 20:1 ratio (specifically, 195 µL of candidate buffer and 5 µL of test sample) in a 96-microwell plate. After reacting for 1 minute, absorbance-related measurements were analyzed at a wavelength of 610 nm, where the maximum peak of the albumin-BCP complex was observed, using a spectrophotometer (Thermo Scientific; Multiskan GO Microplate Spectrophotometer).

Based on the absorbance-related measurements for each candidate buffer under the five pH conditions, linearity (RQS) and slope were calculated.

The five candidate buffers and five pH conditions are as follows Table 1. Table 1 shows five candidate reaction buffers and the pH conditions, for evaluation of albumin measurement performance. For each sample, if the linearity (RQS) was measured to be greater than 0.98 and the slope was 0.003 or higher, the corresponding candidate buffer and pH condition were evaluated as providing sufficient measurement performance for albumin-BCP analysis.

**Table 1: Candidate reaction buffers and the pH conditions, for evaluation of albumin measurement performance.**

| **Buffer** | **HEPES** | **PB** | **Tris-HCl** | **Tris-Base** | **PBS** |
|---|---|---|---|---|---|
| **pH** | pH 4.1 / 5.8 / 6.5 / 8.0 / 10.0 | | | | |

### 2. Experimental Results

Fig. 10 is a table and graph showing the results of evaluating the measurement performance of albumin based on different reaction buffers, according to an embodiment of the present application.

For the HEPES buffer:
1) Under pH 5.8, pH 6.5, and pH 8.0 conditions, the linearity (RQS) was measured to be greater than 0.98, and the slope was measured to be 0.003 or higher.
2) Under pH 4.1 and pH 10.0 conditions, the slope was measured to be less than 0.003.

For the PB buffer:
1) Under pH 5.8, pH 6.5, and pH 8.0 conditions, the linearity (RQS) was measured to be greater than 0.98, and the slope was measured to be 0.003 or higher.
2) Under pH 4.1 and pH 10.0 conditions, the slope was measured to be less than 0.003.

For the Tris-HCl buffer:
1) Under pH 5.8, pH 6.5, and pH 8.0 conditions, the linearity (RQS) was measured to be greater than 0.98, and the slope was measured to be 0.003 or higher.
2) Under pH 4.1 conditions, the linearity (RQS) was measured to be less than 0.98, and under pH 10.0 conditions, the slope was measured to be less than 0.003.

For the Tris-Base buffer:
1) Under pH 4.1, pH 5.8, pH 6.5, and pH 8.0 conditions, the linearity (RQS) was measured to be greater than 0.98, and the slope was measured to be 0.003 or higher.
2) Under pH 10.0 conditions, the slope was measured to be less than 0.003.

For the PBS buffer:
1) Under pH 5.8 and pH 6.5 conditions, the linearity (RQS) was measured to be greater than 0.98, and the slope was measured to be 0.003 or higher.
2) Under pH 4.1, pH 8.0, and pH 10.0 conditions, the slope was measured to be less than 0.003.

Through Experimental Example 1, it was confirmed that the reaction buffer (B) according to an embodiment of the present application may be selected from the group consisting of HEPES, PB, Tris-HCl, Tris-Base, and PBS, and the pH of the first composition containing the reaction buffer (B) may be within the range of 4.1 to 10.0. Preferably, the pH of the first composition containing the reaction buffer (B) may be implemented within the range of 5.8 to 8.0.

### <Experimental Example 2: Evaluation of Glycated Albumin Measurement Performance Based on Reaction Buffers>

### 1. Experimental Method

To select the reaction buffer and reaction conditions (pH) that create the optimal environment for both the BCP analysis method and the enzymatic method for glycated albumin analysis, five candidate buffers (HEPES, Phosphate Buffer, Tris-HCl, Tris-Base, and Phosphate-buffered Saline) were selected. Using these five candidate buffers, the measurement performance of albumin-BCP analysis was evaluated under five different pH conditions (pH 4.1, 5.8, 6.5, 8.0, and 10.0), resulting in a total of 25 buffer-pH combinations. Specifically, each candidate buffer was supplemented with an appropriate concentration of Bromocresol Purple (BCP) reagent and the non-ionic surfactant Triton X-100.

The test samples were prepared by mixing the low-concentration (4.51 g/L) and high-concentration (17.59 g/L) standard solutions of Lucica GA-L Control, a standard solution from Asahi Kasei Pharma used as the reference reagent by i-SENS. Specifically, a medium-concentration sample (11.37 g/L) was prepared by mixing the low-concentration sample and the high-concentration sample of the standard solution at a 1:1 ratio, a mid-low concentration sample (8.01 g/L) was prepared by mixing the low-concentration sample and the medium-concentration sample at a 1:1 ratio, and a mid-high concentration sample (14.66 g/L) was prepared by mixing the medium-concentration sample and the high-concentration sample at a 1:1 ratio.

The prepared samples were verified for glycated albumin (g/L) content using the reference reagent (Lucica GA-L, Asahi Kasei Pharma) and the reference equipment, an automated biochemical analyzer (RX Imola, RANDOX), before use.

Furthermore, the absorbance measurements related to the color development of the chromogenic agent (DA-67) through glycated albumin-enzymatic analysis were analyzed using the A1Care Analyzer from i-SENS (In vitro Diagnostic Approval No. 21-1020), at a wavelength of 660 nm where the maximum peak of the chromogenic agent (DA-67) was observed.

Furthermore, based on the absorbance-related measurements for each candidate buffer under different pH conditions, linearity (RQS) and slope were calculated.

The five candidate buffers and five pH conditions are as follows Table 2. Table 2 shows five candidate reaction buffers and the pH conditions, for evaluation of glycated albumin measurement performance.

For each sample, if the linearity (RQS) was measured to be greater than 0.98 and the slope was 0.0003 or higher, the corresponding candidate buffer and pH condition were evaluated as providing sufficient measurement performance for glycated albumin-enzymatic analysis.

**Table 2: Candidate reaction buffers and the pH conditions, for evaluation of glycated albumin measurement performance.**

| **Buffer** | **HEPES** | **PB** | **Tris-HCl** | **Tris-Base** | **PBS** |
|---|---|---|---|---|---|
| **pH** | pH 4.1 / 5.8 / 6.5 / 8.0 / 10.0 | | | | |

### 2. Experimental Results

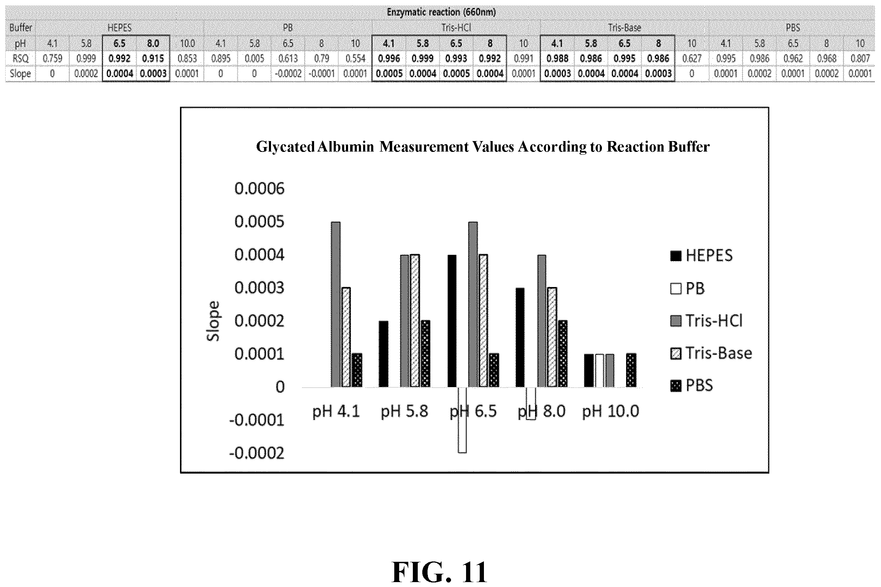

Fig. 11 is a table and graph showing the results of evaluating the measurement performance of glycated albumin based on different reaction buffers, according to an embodiment of the present application.

For the HEPES buffer:
1) Under pH 6.5 and pH 8.0 conditions, the linearity (RQS) was measured to be greater than 0.98, and the slope was measured to be 0.0003 or higher.
2) Under pH 4.1, pH 5.8, and pH 10.0 conditions, the slope was measured to be less than 0.0003.

For the PB buffer, under pH 4.1, pH 5.8, pH 6.5, pH 8.0, and pH 10.0 conditions, the linearity (RQS) was measured to be less than 0.98, and the slope was measured to be less than 0.0003. Notably, for the PB buffer, under pH 5.8 conditions, the slope was practically 0, and under pH 6.5 and pH 8.0 conditions, the slope was practically measured as a negative value. From this, it was confirmed that for the PB buffer, enzymatic reactions do not proceed, or as the concentration of glycated albumin (or glycated amino acids) increases, the signal intensity decreases, indicating that the PB buffer is not suitable for use in glycated albumin-enzymatic analysis.

For the Tris-HCl buffer:
1) Under pH 4.1, pH 5.8, pH 6.5, and pH 8.0 conditions, the linearity (RQS) was measured to be greater than 0.98, and the slope was measured to be 0.0003 or higher.
2) Under pH 10.0 conditions, the slope was measured to be less than 0.0003.

For the Tris-Base buffer:
1) Under pH 4.1, pH 5.8, pH 6.5, and pH 8.0 conditions, the linearity (RQS) was measured to be greater than 0.98, and the slope was measured to be 0.0003 or higher.
2) Under pH 10.0 conditions, the linearity (RQS) was measured to be less than 0.98, and the slope was measured to be less than 0.0003.

For the PBS buffer:
Under pH 4.1, pH 5.8, pH 6.5, pH 8.0, and pH 10.0 conditions, the slope was measured to be less than 0.0003.

Through Experimental Example 2, it was confirmed that the reaction buffer (B) according to an embodiment of the present application may be selected from the group consisting of Tris-HCl and Tris-Base, and the pH of the first composition containing the reaction buffer (B) may fall within the range of 4.1 to 10.0. Preferably, the pH of the first composition containing the reaction buffer (B) may be implemented within the range of 4.1 to 8.0. More preferably, the pH of the first composition containing the reaction buffer (B) may be implemented within the range of 5.8 to 8.0.

Meanwhile, it was observed that under pH 6.5 and pH 8.0 conditions, the slope of the Tris-HCl buffer was relatively larger than that of the Tris-Base buffer. Accordingly, the reaction buffer (B) in a preferred embodiment of the present application may be Tris-HCl. Furthermore, the pH of the first composition containing Tris-HCl as the reaction buffer (B) may be implemented within the range of 4.1 to 10.0. More preferably, the pH of the first composition containing Tris-HCl as the reaction buffer (B) may be implemented within the range of 4.1 to 8.0, and even more preferably, within the range of 5.8 to 8.0.

Meanwhile, the pH of the first composition containing Tris-HCl as the reaction buffer
(B) may be preferably provided in a weakly alkaline pH range, considering the optimal reaction environment for ketoamine oxidase (KAO) of the reaction reagent (R1).

### <Experimental Example 3: Evaluation of Measurement Performance by Surfactants>

### 1. Experimental Method

To select the surfactant that provides the optimal analytical environment for both the BCP analysis method and the enzymatic method, the following evaluations were conducted:
1) The measurement performance of albumin-BCP analysis for different concentrations of each candidate surfactant.
2) The measurement performance of glycated albumin-enzymatic analysis for different concentrations of each candidate surfactant.

Specifically, regarding 1) the measurement performance of albumin-BCP analysis, each candidate surfactant was applied to the albumin samples (Sigma Aldrich) at concentrations of 0.1%, 0.01%, and 0.001%.

The measurement samples were prepared using albumin powder (Albumin from Human Serum, lyophilized powder, Sigma Aldrich; A3782).

Specifically, A low-concentration sample (16.6 g/L) was prepared by dissolving 83 mg of albumin powder in 5 mL of deionized water (DIW), and a high-concentration sample (64.8 g/L) was prepared by dissolving 130 mg of albumin powder in 5 mL of deionized water (DIW), and a medium-concentration sample (40.4 g/L) was prepared by mixing the low-concentration sample and the high-concentration sample in a 1:1 ratio. Furthermore, a mid-low concentration sample (28.4 g/L) was prepared by mixing the low-concentration sample and the medium-concentration sample in a 1:1 ratio, and a mid-high concentration sample (52.6 g/L) was prepared by mixing the medium-concentration sample and the high-concentration sample in a 1:1 ratio, creating a total of five sample concentrations.

The prepared samples were verified for albumin (g/L) values using the reference reagent (Lucica GA-L, Asahi Kasei Pharma) and the reference device, an automatic biochemical analyzer (RX Imola, RANDOX), before use.

Furthermore, using the A1Care Analyzer from i-SENS, the absorbance-related measurement values for each sample were analyzed at 610 nm, where the maximum peak of the BCP complex was observed. Based on the absorbance-related measurements for each candidate surfactant at various concentrations, linearity (RQS) and slope were calculated. For each sample, if the linearity (RQS) was measured to be greater than 0.98 and the slope was 0.003 or higher, the corresponding surfactant and its concentration were evaluated as providing sufficient measurement performance for albumin-BCP analysis.

Furthermore, regarding 2) the measurement performance of glycated albumin-enzymatic analysis, each candidate surfactant was applied to glycated albumin samples (Sigma Aldrich) at concentrations of 0.1%, 0.01%, and 0.001%. The test samples were prepared by mixing the low-concentration (4.51 g/L) and high-concentration (17.59 g/L) standard solutions of Lucica GA-L Control, a standard solution from Asahi Kasei Pharma used as the reference reagent by i-SENS. Specifically, a medium-concentration sample (11.37 g/L) was prepared by mixing the low-concentration and high-concentration standard solutions at a 1:1 ratio, and a mid-low concentration sample (8.01 g/L) was prepared by mixing the low-concentration sample and the medium-concentration sample at a 1:1 ratio, and a mid-high concentration sample (14.66 g/L) was prepared by mixing the medium-concentration sample and the high-concentration sample at a 1:1 ratio.

The prepared samples were verified for glycated albumin (g/L) values using the reference reagent (Lucica GA-L, Asahi Kasei Pharma) and the reference device, an automatic biochemical analyzer (RX Imola, RANDOX), before use.

Furthermore, using the A1Care Analyzer from i-SENS, the absorbance measurements related to the color development of the chromogenic agent (DA-67) through glycated albumin-enzymatic analysis were analyzed for each sample at 660 nm, where the maximum peak of DA-67 was observed. Based on the absorbance-related measurements for each candidate surfactant at various concentrations, linearity (RQS) and slope were calculated. For each sample, if the linearity (RQS) was measured to be greater than 0.98 and the slope was 0.0003 or higher, the corresponding surfactant and its concentration were evaluated as providing sufficient measurement performance for glycated albumin-enzymatic analysis.

The three candidate surfactants and their concentration conditions are as follows Table 3. Table 3 shows candidate surfactants and concentration conditions of surfactants.

**Table 3: Candidate surfactants and Concentration conditions of surfactants.**

| **Surfactants** | **CHAPSO** | | | **Triton X-100** | | | **Tween20** | | |
|---|---|---|---|---|---|---|---|---|---|
| **Concentration** | 0.1% | 0.01% | 0.001% | 0.1% | 0.01% | 0.001% | 0.1% | 0.01% | 0.001% |

### 2. Experimental Results

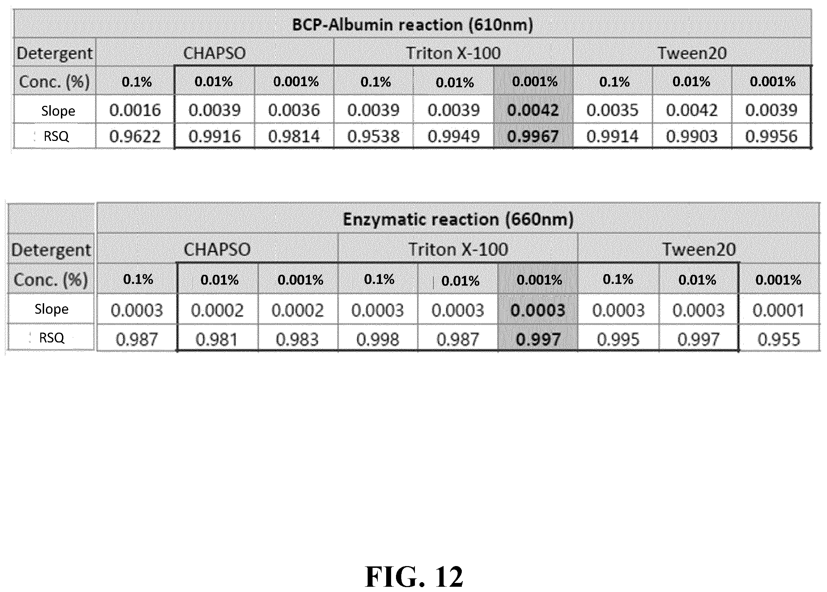

Fig. 12 is a table showing the results of evaluating the measurement performance of albumin and/or glycated albumin based on surfactants added to the reaction buffer, according to an embodiment of the present application.

### 1) Regarding the measurement performance of albumin-BCP analysis

For the CHAPSO surfactant, under 0.01% and 0.001% concentration conditions, the linearity (RQS) was measured to be greater than 0.98, and the slope was measured to be 0.003 or higher. However, under 0.1% concentration conditions, the linearity (RQS) was measured to be less than 0.98, and the slope was measured to be less than 0.003.

For the Triton X-100 surfactant, under 0.1%, 0.01%, and 0.001% concentration conditions, the linearity (RQS) was measured to be greater than 0.98, and the slope was measured to be 0.003 or higher.

For the Tween 20 surfactant, under 0.1%, 0.01%, and 0.001% concentration conditions, the linearity (RQS) was measured to be greater than 0.98, and the slope was measured to be 0.003 or higher.

### 2) Regarding the measurement performance of Glycated albumin-enzymatic analysis

For the CHAPSO surfactant, under 0.1%, 0.01%, and 0.001% concentration conditions, the linearity (RQS) was measured to be greater than 0.98, and the slope was measured to be 0.0003 or higher.

For the Triton X-100 surfactant, under 0.1%, 0.01%, and 0.001% concentration conditions, the linearity (RQS) was measured to be greater than 0.98, and the slope was measured to be 0.0003 or higher.

For the Tween 20 surfactant, under 0.1% and 0.01% concentration conditions, the linearity (RQS) was measured to be greater than 0.98, and the slope was measured to be 0.0003 or higher. However, under 0.1% concentration conditions, the linearity (RQS) was measured to be less than 0.98, and the slope was measured to be less than 0.0003.

Through Experimental Example 3, it was confirmed that the surfactant in an embodiment of the present application may be selected from the group consisting of CHAPSO, Triton X-100, and Tween 20.

Meanwhile, referring to Experimental Example 3, it was confirmed that Triton X-100 satisfies the evaluation criteria under all concentration conditions for both 1) albumin-BCP analysis and 2) glycated albumin-enzymatic analysis. Accordingly, the surfactant in a preferred embodiment of the present application may be Triton X-100, and the concentration of Triton X-100 may be included in the first composition at a concentration exceeding 0% and up to 0.1%.

Furthermore, referring to Experimental Examples 1 to 3, in a preferred embodiment of the present application, the first composition may include Triton X-100 at a concentration exceeding 0% and up to 0.1%, along with a Tris-HCl buffer. This ensures that both the glycated albumin decomposition reaction and the enzymatic reaction of glycated amino acids are carried out in an optimal analytical environment (weakly alkaline). More preferably, the first composition may include Triton X-100 at a concentration of approximately 0.01% and a Tris-HCl buffer.

The second composition, third composition, and fifth composition according to an embodiment of the present application may each include a stabilizer for fixing reagents in a "solid state," as described above. Each stabilizer may include disaccharides, DEAE-Dextran, and NPS.

To fix the reaction reagent (R1), decomposition reagent (R2), and oxidase (E) in a "solid state," a drying process is required. During this drying process, the rapid removal of moisture may cause structural damage to the proteins constituting the reagents. To prevent such structural damage, disaccharides were selected as one of the stabilizers. Specifically, trehalose which is a disaccharide, was selected as one of the stabilizers.

In the dried state, the reaction reagent (R1), decomposition reagent (R2), and oxidase (E) must maintain stability for several months or even years. To ensure storage stability, NPS (Neo Protein Saver) was selected as one of the stabilizers, as it interacts with the protein molecules in the reagents to protect their structure and prevent protein denaturation caused by oxidation.

In the dried state, reaction reagent (R1), decomposition reagent (R2), and oxidase (E) must also be able to re-dissolve and participate in reactions when mixed with the blood sample. To facilitate this, DEAE-Dextran was selected as one of the stabilizers, as it protects protein structures through ion exchange and aids in the re-dissolution of dried reagents.

### <Experimental Example 4: Evaluation of Accelerated Stability by Stabilizers>

### 1. Experimental Method

The A1Care Cartridge from i-SENS (In vitro Diagnostic Approval No. 21-4641, 3 lots), in which the present invention is implemented, was used.

A stabilizer composed of 250 mM trehalose, 1.25 (w/v%) DEAE-dextran, and 1.5 (w/v%) NPS (TOYOBO) was added to the reagent fixing part of the A1Care Cartridge, where the reaction reagent (R1), consisting of KAO and POD, was immobilized.

A stabilizer composed of 250 mM trehalose, 1.25 (w/v%) DEAE-dextran, and 1.5 (w/v%) NPS (TOYOBO) was added to the reagent fixing part of the A1Care Cartridge, where the oxidase (E), consisting of ASOx, was immobilized.

A stabilizer composed of 250 mM trehalose, 1.25 (w/v%) DEAE-dextran, and 1.5 (w/v%) NPS (TOYOBO) was added to the reagent fixing part of the A1Care Cartridge, where the decomposition reagent (R2), consisting of protease, was immobilized.

Meanwhile, 100 mM trehalose was added to the reagent fixing part of the A1Care Cartridge, where the chromogenic agent (D), consisting of DA-67, was immobilized.

The test samples were prepared using the standard solution (Lucica GA-L Control) from Asahi Kasei Pharma, which is utilized as the i-SENS's reference reagent. The low-concentration (4.51 g/L) and high-concentration (17.59 g/L) solutions were verified for albumin (g/L) and glycated albumin (g/L and %) values using the reference reagent (Lucica GA-L, Asahi Kasei Pharma) and the reference device, an automated biochemical analyzer (RX Imola, RANDOX), prior to use. The glycated albumin ratio (GA%) was calculated using the A1Care Analyzer from i-SENS (2 Lots).

For one lot of the A1Care Analyzer (2 Lots), the Lucica GA-L Control Solution (Low) was used to perform 10 repeated measurements on evaluation days for 3 lots of cartridges. For the remaining one lot of the A1Care Analyzer, the Lucica GA-L Control Solution (High) was used to perform 10 repeated measurements on evaluation days for 3 lots of cartridges to measure the glycated albumin ratio (GA%).

Additionally, the bias (%) of the measured glycated albumin ratios relative to the initial glycated albumin ratio (GA%) and the precision (CV%) were calculated.

The above analysis method was performed for each of the following storage conditions in Table 4 below. Table 4 shows the storage temperature and storage period of the cartridge.

**Table 4: Storage temperature and storage period of the cartridge**

| **Storage temperature of cartridge** | **2 ~ 8 °C** | **20** ~ **25 °C** | **40 °C** |
|---|---|---|---|
| **Storage period** | up to 52 weeks | up to 52 weeks | up to 2 weeks |

Furthermore, a stabilizer was evaluated as suitable for the quantitative analysis of the glycated albumin ratio (GA%) if it satisfied all of the following evaluation criteria:
1) Among 3 cartridge lots, at least 2 lots must satisfy the following conditions for the measurement value of the initial glycated albumin ratio (i.e., GA% on day 0). The bias must be within ±10%, and the CV must be within 10%.
2) Additionally, at each evaluation period, the average measurement value of the glycated albumin ratio for the 3 cartridge lots must satisfy the following conditions. The bias must be within ±10%, and the CV must be within 10%.

### 2. Experimental Results

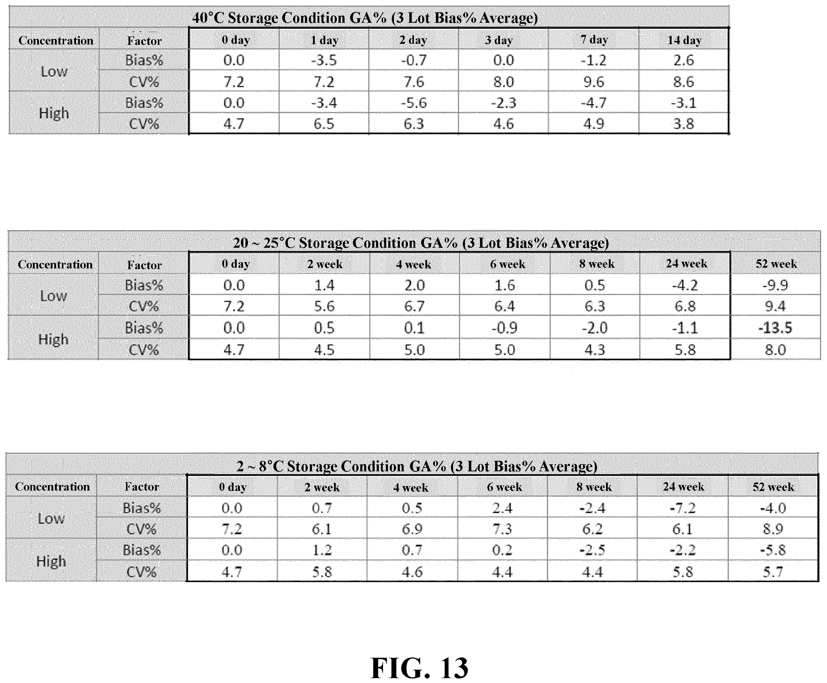

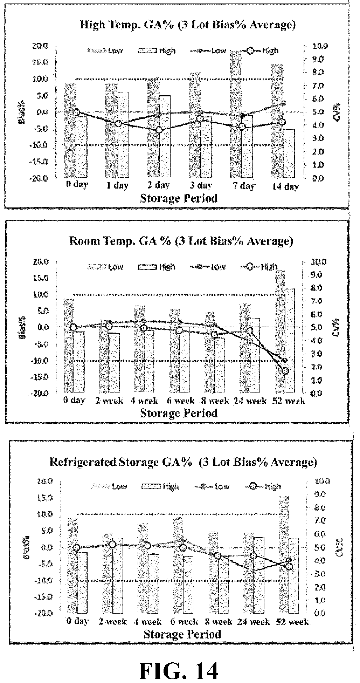

Fig. 13 is a table showing the results of evaluating the accelerated stability of reaction reagents, oxidases, and/or decomposition reagents based on stabilizers added thereto, according to an embodiment of the present application. Fig. 14 is a graph showing the results of evaluating the accelerated stability of reaction reagents, oxidases, and/or decomposition reagents based on stabilizers added thereto, according to an embodiment of the present application.

Under the storage condition of 40°C, it was confirmed that the average measurement value of the glycated albumin ratio for the 3 cartridge lots remained within a bias of ±10% and a CV of 10% until 14 days of storage.

Under the storage condition of 20-25°C, it was confirmed that the average measurement value of the glycated albumin ratio for the 3 cartridge lots remained within a bias of ±10% and a CV of 10% until 24 weeks of storage.

Under the storage condition of 2-8°C, it was confirmed that the average measurement value of the glycated albumin ratio for the 3 cartridge lots remained within a bias of ±10% and a CV of 10% until 52 weeks of storage.

Through Experimental Example 4, the stabilizer containing disaccharides, DEAE-Dextran, and NPS, each added to the second composition, third composition, and fifth composition, was evaluated as suitable for the quantitative analysis of the glycated albumin ratio.

Meanwhile, in Experimental Example 4, trehalose was used as the disaccharide included in the stabilizer. However, any suitable disaccharide with characteristics similar to trehalose, such as sucrose, may also be included in the stabilizer.

### <Experimental Example 5: Equivalency of Trehalose and Sucrose as Stabilizers>

To verify that disaccharides other than trehalose can be used as stabilizers, the equivalency of trehalose and sucrose, a representative disaccharide, as stabilizers was additionally tested.

### 1. Experimental Method

To verify that disaccharides can be used as stabilizers, a comparative evaluation was conducted as shown in Table 5 below. Table 5 shows the compositions of the two stabilizers used in the comparative evaluation. The evaluation compared two stabilizer formulations: 1) Stabilizer Condition 1, composed of trehalose, DEAE-dextran at 1.25% w/v, and NPS at 1.5% w/v, and 2) Stabilizer Condition 2, composed of sucrose, DEAE-dextran at 1.25% w/v, and NPS at 1.5% w/v.

**Table 5: Compositions of the two stabilizers used in the comparative evaluation**

| **Reagent Stabilizer** | **Condition #1** | **Condition #2** |
|---|---|---|
| **Trehalose** | 250 mM | - |
| **Sucrose** | - | 250 mM |
| **DEAE-Dextran** | 1.25% w/v | 1.25% w/v |
| **NPS** | 1.50% w/v | 1.50% w/v |

First, the measurement values of glycated albumin, total albumin, and the glycated albumin ratio when using Stabilizer of Condition 1 containing trehalose were compared with the measurement values of glycated albumin, total albumin, and the glycated albumin ratio when using Stabilizer of Condition 2 containing sucrose. Furthermore, the precision of the measurement values (i.e., the measurement values of glycated albumin, total albumin, and the glycated albumin ratio) when using Stabilizer of Condition 1 containing trehalose was measured and compared with the precision of the measurement values (i.e., the measurement values of glycated albumin, total albumin, and the glycated albumin ratio) when using Stabilizer of Condition 2 containing sucrose.

The samples used for the comparison of measurement values and the evaluation of precision were prepared using the low-concentration and high-concentration standard solutions (Lucica GA-L Control) from Asahi Kasei Pharma. The prepared samples were confirmed for glycated albumin (g/L) and total albumin (g/L) values using the reference reagent (Lucica GA-L, Asahi Kasei Pharma) and the reference device, an automated biochemical analyzer (RX Imola, RANDOX), and the final glycated albumin ratio (%) values were calculated based on these two values prior to use. Furthermore, the A1Care Analyzer (1 Lot) from i-SENS and A1Care GA Cartridges (1 Lot each) containing each of the stabilizers of Condition 1 and Condition 2, respectively, were used to perform 33 repeated measurements of the standard solution samples at each concentration. Based on these measurements, the glycated albumin (GA g/L) values, total albumin (ALB g/L) values, glycated albumin ratio (GA%) values, and the precision of each measurement value were calculated.

Secondly, the linearity of each measurement value (i.e., the glycated albumin measurement value, total albumin measurement value, and glycated albumin ratio measurement value) obtained using stabilizer of Condition 1 containing trehalose was evaluated. Similarly, the linearity of each measurement value (i.e., the glycated albumin measurement value, total albumin measurement value, and glycated albumin ratio measurement value) obtained using the stabilizer of Condition 2 containing sucrose was also evaluated.

The measurement samples for evaluating the linearity of total albumin were prepared using albumin powder (Albumin from Human Serum Albumin lyophilized powder, Sigma Aldrich; A3782). Specifically, 83 mg of albumin powder was dissolved in 5 mL of deionized water (DIW) to prepare a low-concentration sample (16.6 g/L), and 130 mg of albumin powder was dissolved in 5 mL of DIW to prepare a high-concentration sample (64.8 g/L). And a medium-concentration sample (40.4 g/L) was prepared by mixing the low- and high-concentration samples in a 1:1 ratio. Furthermore, a low-medium concentration sample (28.4 g/L) was prepared by mixing the low-concentration sample and the medium-concentration sample in a 1:1 ratio, and a medium-high concentration sample (52.6 g/L) was prepared by mixing the medium-concentration sample and the high-concentration sample in a 1:1 ratio, resulting in a total of five concentration samples. The prepared samples were verified for albumin (g/L) values using the reference reagent (Lucica GA-L, Asahi Kasei Pharma) and the reference device, an automated biochemical analyzer (RX Imola, RANDOX), prior to use.

The measurement samples for evaluating the linearity of glycated albumin were prepared by mixing the low-concentration solution (5.96 g/L) and high-concentration solution (15.41 g/L) of the standard solution (Lucica GA-L Control) from Asahi Kasei Pharma. Specifically, a medium-concentration sample (10.836 g/L) was prepared by mixing the low-concentration and high-concentration standard solutions in a 1:1 ratio. Furthermore, a low-medium concentration sample (8.41 g/L) was prepared by mixing the low-concentration sample and the medium-concentration sample in a 1:1 ratio, and a medium-high concentration sample (15.41 g/L) was prepared by mixing the medium-concentration sample and the high-concentration sample in a 1:1 ratio. The prepared samples were verified for glycated albumin (g/L) values using the reference reagent (Lucica GA-L, Asahi Kasei Pharma) and the reference device, an automated biochemical analyzer (RX Imola, RANDOX), prior to use.

Furthermore, the i-SENS A1Care Analyzer (In Vitro Diagnostic Approval No. 21-1020) was used to analyze the measurement values related to absorption related to the coloration of the chromogenic agent (DA-67) resulting from glycated albumin-enzyme analysis at the wavelength of 660 nm, where the maximum peak of the chromogenic agent (DA-67) was observed. Additionally, the measurement values related to changes in absorbance from the total albumin-BCP analysis were analyzed at the wavelength of 610 nm, where the maximum peak of total albumin and BCP reagent was observed.

Furthermore, for each of the stabilizer of Condition 1 and stabilizer of Condition 2, the RSQ values, which indicate the linearity of measurement values depending on sample concentration (i.e., the measurement values of glycated albumin, total albumin, and the glycated albumin ratio), were calculated.

Additionally, if the following evaluation criteria were all satisfied, stabilizer of Condition 1 containing trehalose and stabilizer of Condition 2 containing sucrose were assessed to exhibit equivalent performance as stabilizers for the quantitative analysis of glycated albumin.
1) The measurement values for the stabilizer of Condition 1 containing trehalose and stabilizer of Condition 2 containing sucrose must satisfy a Bias% within the range of -10% to 10%.
2) The precision (CV%) of the measurement values for stabilizer of Condition 1 containing trehalose and stabilizer of Condition 2 containing sucrose must be within 5%.
3) The linearity (RSQ) of the measurement values for stabilizer of Condition 1 containing trehalose and stabilizer of Condition 2 containing sucrose must be 0.98 or greater.

### 2. Experimental Results

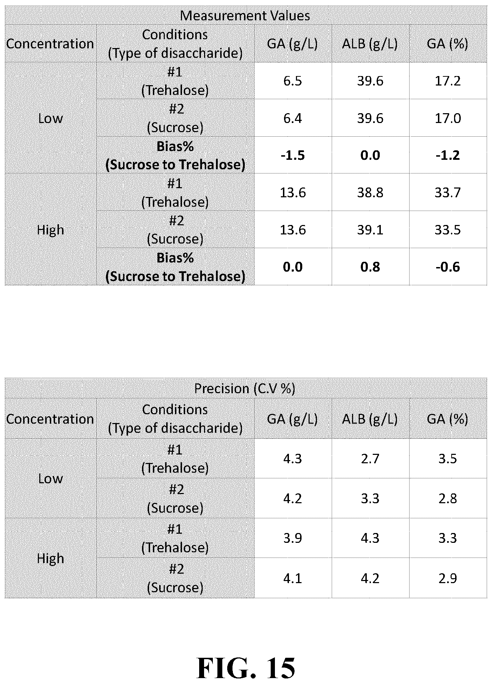

Fig. 15 is a table showing the measurements and precision based on the type of disaccharide included in the stabilizers added to the reaction reagents, oxidases, and/or decomposition reagents, according to an embodiment of the present application.

For the low-concentration (Low) standard solution sample, in the case of the stabilizer of Condition 1 containing trehalose, the measurement value of glycated albumin (GA) was 6.5, and in the case of the stabilizer of Condition 2 containing sucrose, the measurement value of glycated albumin (GA) was 6.4, with a Bias(%) of -1.5%. And in the case of the stabilizer of Condition 1 containing trehalose, the measurement value of total albumin (ALB) was 39.6, and in the case of the stabilizer of Condition 2 containing sucrose, the measurement value of total albumin (ALB) was also 39.6, with a Bias(%) of 0%. And in the case of the stabilizer of Condition 1 containing trehalose, the measurement value of glycated albumin ratio (GA(%)) was 17.2, and in the case of the stabilizer of Condition 2 containing sucrose, the measurement value of glycated albumin ratio (GA(%)) was 17.0, with a Bias(%) of -1.2%.

For the high-concentration (High) standard solution sample, in the case of the stabilizer of Condition 1 containing trehalose, the measurement value of glycated albumin (GA) was 13.6, and in the case of the stabilizer of Condition 2 containing sucrose, the measurement value of glycated albumin (GA) was also 13.6, with a Bias(%) of 0%. And in the case of the stabilizer of Condition 1 containing trehalose, the measurement value of total albumin (ALB) was 38.8, and in the case of the stabilizer of Condition 2 containing sucrose, the measurement value of total albumin (ALB) was 39.1, with a Bias(%) of 0.8%. And in the case of the stabilizer of Condition 1 containing trehalose, the measurement value of glycated albumin ratio (GA(%)) was 33.7, and in the case of the stabilizer of Condition 2 containing sucrose, the measurement value of glycated albumin ratio (GA(%)) was 33.5, with a Bias(%) of -0.6%.

Thus, it was confirmed that the measurement values of the stabilizer of Condition 1 and the stabilizer of Condition 2 satisfied the evaluation criterion, which requires that the Bias(%) for all cases falls within the range of -10% to 10%.

For the low concentration (Low) standard solution sample, when using the stabilizer of Condition 1 containing trehalose, the precision (CV%) of the glycated albumin (GA) measurement value was 4.3, the precision (CV%) of the total albumin (ALB) measurement value was 2.7, and the precision (CV%) of the glycated albumin ratio (GA(%)) was 3.5. When using the stabilizer of Condition 2 containing sucrose, the precision (CV%) of the glycated albumin (GA) measurement value was 4.2, the precision (CV%) of the total albumin (ALB) measurement value was 3.3, and the precision (CV%) of the glycated albumin ratio (GA(%)) was 2.8.

For the high concentration (High) standard solution sample, when using the stabilizer of Condition 1 containing trehalose, the precision (CV%) of the glycated albumin (GA) measurement value was 3.9, the precision (CV%) of the total albumin (ALB) measurement value was 4.3, and the precision (CV%) of the glycated albumin ratio (GA(%)) was 3.3. When using the stabilizer of Condition 2 containing sucrose, the precision (CV%) of the glycated albumin (GA) measurement value was 4.1, the precision (CV%) of the total albumin (ALB) measurement value was 4.2, and the precision (CV%) of the glycated albumin ratio (GA(%)) was 2.9.

Thus, it was confirmed that the precision (CV) of the measurement values for the stabilizer of Condition 1 and the stabilizer of Condition 2 met the evaluation criterion of being within 5% in all cases.

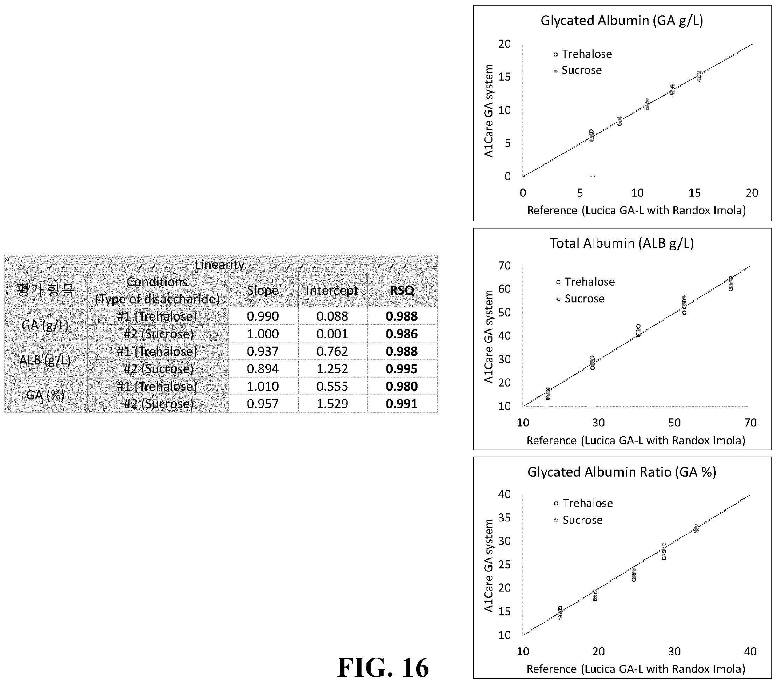

Fig. 16 is a table and graph showing the results of evaluating linearity based on the type of disaccharide included in the stabilizers added to the reaction reagents, oxidases, and/or decomposition reagents, according to an embodiment of the present application.

As a result of the linearity evaluation, the indicator (RSQ) representing the linearity of the glycated albumin (GA) measurement value when using the stabilizer of Condition 1, which includes trehalose, was measured at 0.988. And the indicator (RSQ) for the linearity of the total albumin (ALB) measurement value was measured at 0.988, and the indicator (RSQ) for the linearity of the glycated albumin ratio (GA%) measurement value was measured at 0.980.

Furthermore, when using the stabilizer of Condition 2, which includes sucrose, the indicator (RSQ) for the linearity of the glycated albumin (GA) measurement value was measured at 0.986, the indicator (RSQ) for the linearity of the total albumin (ALB) measurement value was measured at 0.995, and the indicator (RSQ) for the linearity of the glycated albumin ratio (GA%) measurement value was measured at 0.991.

Thus, it was confirmed that the linearity (RSQ) of the measurement values for the stabilizers in both Condition 1 and Condition 2 satisfied the evaluation criterion of being at least 0.98 in all cases.

Through Experimental Example 5, it was confirmed that both the stabilizer of Condition 1, containing trehalose, and the stabilizer of Condition 2, containing sucrose, can be used as stabilizers for the quantitative analysis of glycated albumin. Furthermore, through Experimental Example 5, it was additionally confirmed that disaccharides exhibiting similar characteristics to trehalose and sucrose can sufficiently perform as stabilizers for the quantitative analysis of glycated albumin.

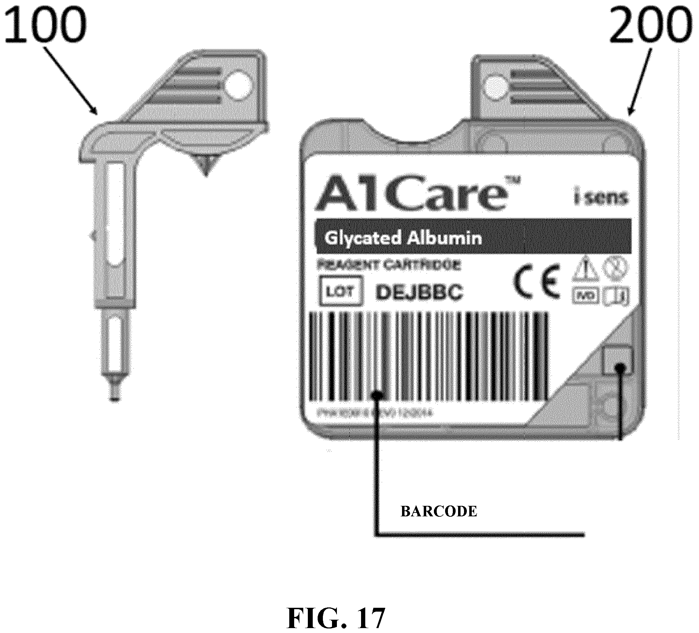

Fig. 17 is a diagram showing the identification information included in the glycated albumin quantitative analysis kit according to an embodiment of the present application.

The GA quantitative analysis kit (10) according to one embodiment of the present application may include identification information for recognizing a biological sample. For example, the GA quantitative analysis kit (10) may include identification information (e.g., information in the form of a barcode) on one surface of the main cartridge (200) to identify the type of biological sample.

The quantitative analysis device, described later, may acquire identification information to recognize the type of biological sample to be analyzed by the GA quantitative analysis kit (10). For instance, the quantitative analysis device may determine, based on a barcode, that the biological sample for analysis is GA. In this case, the quantitative analysis device may be configured to perform quantitative analysis of the biological sample by executing a pre-stored analysis protocol associated with the recognized type, based on the recognized type of the biological sample. For example, the quantitative analysis device may be implemented to execute a pre-stored GA analysis protocol based on recognizing that the type of biological sample to be analyzed is GA, thereby performing quantitative analysis of GA.

Meanwhile, FIG 17 illustrates identification information in the form of a barcode as an example. However, this is merely an example, and any suitable form of identification information may be provided at any suitable location on the GA quantitative analysis kit (10).

The quantitative analysis device (or quantitative analysis apparatus) according to an embodiment of the present disclosure may be configured to perform quantitative analysis of biological samples (e.g., 1,5-AG, glycated albumin(GA), CRP, etc.).

Furthermore, the quantitative analysis device may include a communication module (which may also be referred to as a transceiver), a memory, and/or a processor.

The communication module of the quantitative analysis device may communicate with any external device or external server. For example, the quantitative analysis device may transmit quantitative analysis results to an external device or external server via the communication module.

The quantitative analysis device may access a network through the communication module to transmit or receive various types of data. The communication module may largely include a wired-type communication module and a wireless-type communication module. Since the wired-type communication module and the wireless-type communication module have their own advantages and disadvantages, in some cases, the wired-type communication module and the wireless-type communication module may be provided together as the quantitative analysis device. Here, in the case of the wireless-type communication module, a wireless local area network (WLAN) type communication method such as Wi-Fi may be mainly used. Alternatively, in the case of the wireless-type communication module, cellular communication, such as long-term evolution (LTE) or 5G communication methods, may be used. However, a wireless communication protocol is not limited to the above-described example, and any appropriate wireless type of communication method may be used. In the case of the wired-type communication module, a local area network (LAN) or Universal Serial Bus (USB) communication is a representative example, but other methods may be used.

Various types of information may be stored in the memory of the quantitative analysis device. Various types of data may be temporarily or semi-permanently stored in the memory. Examples of the memory may include a hard disk drive (HDD), a solid state drive (SSD), a flash memory, a read-only memory (ROM), a random access memory (RAM), etc. The memory may be provided to be embedded in the e quantitative analysis device or in a detachable form. Various types of data necessary for the operation of the quantitative analysis device, including an operating program (OS) for driving the quantitative analysis device or a program for operating each component of the quantitative analysis device, may be stored in the memory.

The processor may control the overall operation of the quantitative analysis device. For example, the quantitative analysis device may control its overall operations, including actions such as recognizing the identification information of a biological sample, executing a corresponding analysis protocol based on the recognized identification information, and/or performing quantitative analysis of the biological sample according to the analysis protocol. Specifically, the processor may load and execute a program for the overall operation of the quantitative analysis device from the memory. The processor may be implemented as an application processor (AP), a central processing unit (CPU), a microcontroller unit (MCU), or a similar device depending on hardware, software, or a combination thereof. In this case, the processor may be provided in the form of an electronic circuit that processes electrical signals and performs a control function in hardware, and may be provided in the form of a program or code that drives a hardware circuit in software.

According to an embodiment of the present application, the glycated albumin quantitative analysis kit, the glycated albumin quantitative analysis method, and/or the device for performing the method can provide the effect of implementing both the BCP reaction for quantifying the total amount of albumin and the enzymatic reaction for quantifying the amount of glycated albumin on a single kit through the optimal combination of a reaction buffer and a surfactant.

According to an embodiment of the present application, by implementing the BCP reaction and the enzymatic reaction on a single kit, the glycated albumin quantitative analysis kit can be miniaturized, the analysis time of glycated albumin can be reduced, and the effect of increasing user convenience can be provided.

According to an embodiment of the present application, by immobilizing a composition containing a decomposition reagent onto an appropriate reagent fixing part, the loss of the BCP binding site present in albumin can be prevented, thereby improving the accuracy of the quantification of total albumin using the BCP analysis method.

Effects of the present invention are not limited to the above-described effects and other effects that are not described may be clearly understood by those skilled in the art from the above detailed descriptions.

Features, structures, and effects described in the above-described exemplary embodiments are included in at least one exemplary embodiment of the present invention, but are not necessarily limited to only one exemplary embodiment. Furthermore, features, structures, and effects described in each embodiment can be combined or modified and implemented in other embodiments by one of ordinary skill in the art to which the embodiments belong. Therefore, it should be interpreted that contents related to such combinations and modifications are included in the scope of the present invention.

Further, while the present invention has been particularly described with reference to embodiments, the embodiments are only exemplary embodiments of the present invention and the present invention is not intended to be limited thereto. It will be understood by those skilled in the art that modifications and applications in other forms may be made without departing from the spirit and scope of the present invention. That is, each element specifically shown in the embodiments may be modified and embodied. In addition, it should be understood that differences related to these modifications and applications are within the scope of the present invention as defined in the appended claims.

## Claims

1. A glycated albumin (GA) quantitative analysis kit, comprising:
a first composition including a reaction buffer for inducing binding with albumin present in a blood sample;
a second composition including a decomposition reagent for a decomposition reaction to break down glycated albumin (GA) included in the blood sample into glycated amino acids;
a third composition including a reaction reagent for an enzymatic reaction of the glycated amino acids present in the blood sample; and
a fourth composition including a chromogenic agent being oxidized by hydrogen peroxide (H₂O₂) generated from the enzymatic reaction of the glycated amino acids,
wherein the GA quantitative analysis kit includes at least one reagent fixing part, and
the second composition to the fourth composition are each independently fixed to the reagent fixing part.

2. The GA quantitative analysis kit according to claim 1,
wherein the first composition is selected from the group consisting of Bromocresol Purple (BCP) and Bromocresol Green (BCG).

3. The GA quantitative analysis kit according to claim 2,
wherein the reaction buffer is selected from the group consisting of HEPES, PB, Tris-HCl, Tris-Base, and PBS.

4. The GA quantitative analysis kit according to claim 2,
wherein the reaction buffer is selected from the group consisting of Tris-HCl and Tris-Base,
and the pH of the first composition is in the range of 4.1 to 10.0.

5. The GA quantitative analysis kit according to claim 2,
wherein the reaction buffer is Tris-HCl,
and the decomposition reaction of glycated albumin and the enzymatic reaction of glycated amino acids are performed under a slightly alkaline pH condition.

6. The GA quantitative analysis kit according to claim 3,
wherein the first composition further includes a surfactant selected from the group consisting of CHAPSO, Triton X-100, and Tween 20.

7. The GA quantitative analysis kit according to claim 3,
wherein the first composition further includes Triton X-100 surfactant at a concentration of more than 0% and up to 0.1%.

8. The GA quantitative analysis kit according to claim 2,
wherein the reaction buffer includes Tris-HCl, the first composition further includes Triton X-100 surfactant at a concentration of more than 0% and up to 0.1%,
and the decomposition reaction of glycated albumin and the enzymatic reaction of glycated amino acids are performed under a slightly alkaline pH condition.

9. The GA quantitative analysis kit according to claim 1,
wherein the decomposition reagent is proteinase.

10. The GA quantitative analysis kit according to claim 1,
wherein the reaction reagent includes ketoamine oxidase (KAO) and peroxidase (POD).

11. The GA quantitative analysis kit according to Claim 1,
wherein the chromogenic agent is DA-67.

12. The GA quantitative analysis kit according to claim 1,
wherein the GA quantitative analysis kit further includes a fifth composition comprising an oxidase to prevent interference from ascorbic acid present in the blood sample,
and the oxidase includes ascorbate oxidase (ASOx).

13. The GA quantitative analysis kit according to claim 1,
wherein the second composition further includes a stabilizer for immobilizing the decomposition reagent onto the at least one reagent fixing part in solid form,
and the stabilizer includes disaccharides, DEAE-Dextran, and NPS.

14. The GA quantitative analysis kit according to claim 1,
wherein the third composition further includes a stabilizer for immobilizing the reaction reagent onto the at least one reagent fixing part in solid form,
and the stabilizer includes disaccharides, DEAE-Dextran, and NPS.

15. The GA quantitative analysis kit according to claim 1,
wherein the fourth composition further includes a stabilizer for immobilizing the chromogenic agent onto the at least one reagent fixing part in solid form,
and the stabilizer includes disaccharides.

16. The GA quantitative analysis kit according to claim 12,
wherein the fifth composition further includes a stabilizer for immobilizing ascorbate oxidase onto the at least one reagent fixing part in solid form,
and the stabilizer includes disaccharides, DEAE-Dextran, and NPS.

17. The GA quantitative analysis kit according to claim 12,
wherein the third composition is immobilized in solid state onto a first reagent fixing part located in the first region of the GA quantitative analysis kit,
and the second composition is immobilized in solid state onto a second reagent fixing part located in the second region, which is partitioned from the first region, of the GA quantitative analysis kit.

18. The GA quantitative analysis kit according to claim 17,
wherein the GA quantitative analysis kit is configured such that the reaction buffer is mixed with the reaction reagent fixed to the first reagent fixing part before being mixed with the decomposition reagent fixed to the second reagent fixing part,
and the reaction reagent is **characterized by** performing the enzymatic reaction with the glycated amino acids from the point when the glycated amino acids are generated by the decomposition reaction caused by the decomposition reagent.

19. The GA quantitative analysis kit according to claim 17,
wherein the GA quantitative analysis kit comprises a body including an upper plate and a lower plate configured in a facing arrangement,
the third composition is fixed to the first reagent fixing part located in the first region of the upper plate,
and the fifth composition is fixed to the third reagent fixing part located in the first region of the lower plate,
with the first reagent fixing part and the third reagent fixing part configured to face each other.

20. The GA quantitative analysis kit according to claim 19,
wherein the second composition is fixed to the second reagent fixing part located in the second region of the upper plate,
and the fourth composition is fixed to the fourth reagent fixing part located in the second region of the lower plate,
with the second reagent fixing part and the fourth reagent fixing part configured to face each other.
